(19) Europäisches Patentamt
European Patent Office
Office européen des brevets

(11) Veröffentlichungsnummer: **0 050 850**
**B1**

(12) # EUROPÄISCHE PATENTSCHRIFT

(45) Veröffentlichungstag der Patentschrift: **22.04.87**

(51) Int. Cl.[4]: **C 07 D 209/42**, C 07 D 491/056, A 61 K 31/40

(21) Anmeldenummer: **81108762.6**

(22) Anmeldetag: **23.10.81**

(54) **1-Carboxyalkanoylindolin-2-carbonsäuren und deren Derivate, Verfahren zu ihrer Herstellung, und pharmazeutische Präparate, die diese Verbindungen enthalten.**

(30) Priorität: **27.10.80 US 200706**
**17.02.81 US 235294**

(43) Veröffentlichungstag der Anmeldung:
**05.05.82 Patentblatt 82/18**

(45) Bekanntmachung des Hinweises auf die Patenterteilung:
**22.04.87 Patentblatt 87/17**

(84) Benannte Vertragsstaaten:
**AT BE CH DE FR IT LI LU NL SE**

(56) Entgegenhaltungen:
**EP - A - 0 031 741**
**US - A - 3 780 062**
**US - A - 3 796 723**
**US - A - 3 974 145**
**US - A - 4 052 511**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Gruenfeld, Norbert, Dr., 19 Sparrow Circle, White Plains New York 10605 (US)**

(74) Vertreter: **Zumstein, Fritz, Dr. et al, Bräuhausstrasse 4, D-8000 München 2 (DE)**

**EP 0 050 850 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

1-Alkanoylindolin-2-carbonsäuren und ihre 5,6-Dihydroxy-Derivate, nämlich die N-acylierten Cyclodopaderivate sind in Nippon Kagaku Zasshi 87, 760 (1966), im US-A-3 796 723 bzw. in Helv. Chim. Acta 53, 1701 (1970), z.B. als Beispiele für die Synthese von O- und/oder N-Acylverbindungen, beschrieben. Auch 1-Carboxyacyl-(azetidin, pyrrolidin oder piperidin)-2-carbonsäuren und ihre funktionellen Derivate sind, z.B. aus der US-A-4 052 511, als Verbindungen mit antihypertensiver Wirkung, bekannt. Indolin-Derivate mit antihypertensiver Wirkung sind ferner aus US-A-3 780 062 bekannt.

Es wurde nun überraschend gefunden, dass entweder durch die Einführung einer Carboxygruppe in die vorher genannten Indoline, oder durch Erweiterung der obigen Pyrrolidine zu Indolin-Ringsystemen, wertvolle antihypertensive Mittel erhalten werden.

Indolinringsysteme werden auch vom Gegenstand der älteren Anmeldung EP-A-31 741 umfasst; zwar überlappt dieser mit dem Gegenstand der vorliegenden Erfindung, EP-A-31 741 ist jedoch nicht neuheitschädlich im Sinne von Art 54/3EPÜ.

Die vorliegende Erfindung betrifft die 1-Carboxy-(alkanoyl oder aralkanyol)-indolin-2-carbonsäuren der allgemeinen Formel I,

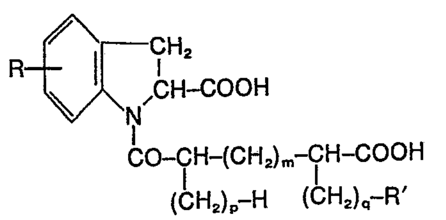

(I)

worin R Wasserstoff, $C_{1-4}$-Alkyl oder $C_{1-4}$-Alkoxy, Halogen oder Trifluormethyl bedeutet, m für die Zahl 0 oder 1 steht, jedes der Symbole p und q eine Zahl von 0 bis 2 bedeutet, und R′ für Wasserstoff oder Phenyl substituiert durch R steht; ihre Mono- oder Bisamide, die Mono- oder Bis-($C_{1-7}$-alkyl oder ω-amino-$C_{2-7}$-alkyl)-ester und ihre Salze, insbesondere ihre therapeutisch verwendbaren Salze, sowie Verfahren zu ihrer Herstellung, pharmazeutische Präparate die diese Verbindungen enthalten, sowie die Verbindungen zur therapeutischen Verwendung.

Die Erfindung betrifft ferner die folgenden Einzelverbindungen welche von Formel I nicht umfasst werden:

1-(4-Carboxy-3-methylbutanoyl)indolin-2S-carbonsäureäthylester, 1-(4-Carboxy-3-methylbutanoyl)-indolin-2S-carbonsäure, 1-(4-Carboäthoxy-2R-isopropyl-butanoyl-indolin-2S-carbonsäure, 1-(4-Carboxy-2-isopropyl-butanoyl)-indolin-2S-carbonsäure, 1-(4-Carboxy-3,3-dimethyl-butanoyl)-indolin-2S-carbonsäure sowie deren Salze.

Die Substituenten R können durch die folgenden Gruppen illustriert werden: $C_{1-4}$-alkyl, z.B. Methyl, Äthyl, n- oder iso-Propyl oder -Butyl; $C_{1-4}$-alkoxy, z.B. Methoxy, Äthoxy, n- oder iso-Propoxy oder -Butoxy; Halogen, z.B. Fluor, Chlor oder Brom; oder Trifluormethyl.

Unter den «$C_{1-7}$-Alkylresten» sind diejenigen mit höchstens 4, insbesondere 1 oder 2 Kohlenstoffatomen bevorzugt.

Die genannten funktionellen Derivate, in welchen entweder eine oder die beiden Carboxygruppen verestert oder amidiert sind, sind vorzugsweise Mono- oder Bis-$C_{1-7}$-alkylester, z.B. Methyl-, Äthyl-, n- oder iso-Propyl- oder -Butylester; oder Mono- oder Bis-amide. Die genannten ω-amino-$C_2$-$C_7$ alkylestern sind vorzugsweise Halbester mit einer freien Indolin-2-carboxygruppe, z.B. die ω-Amino-(äthyl, propyl oder butyl)-ester.

Salze sind vorzugsweise therapeutisch verwendbare Salze, z.B. Metall- oder Ammoniumsalze der genannten Säuren, insbesondere Alkalimetall- oder Erdalkalimetallsalze, z.B. Natrium-, Kalium-, Magnesium oder Calciumsalze; in erster Linie leicht kristallisierende Ammoniumsalze. Diese werden abgeleitet von Ammoniak oder organischen Aminen, z.B. Mono-, Di- oder Tri-$C_{1-7}$-(alkyl, cycloalkyl oder hydroxyalkyl)-aminen, $C_{1-7}$alkylendiaminen oder (Hydroxy-$C_{1-7}$-alkyl oder Aryl-$C_{1-1}$-alkyl)-$C_{1-7}$-alkylammonium Basen, z.B. Methylamin, Di-äthylamin, Triäthylamin, Dicyclohexylamin, Triäthanolamin, Äthylendiamin, Tris-(hydroxymethyl)-aminomethan oder Benzyltrimethylammoniumhydroxid. Die genannten basischen ω-Amino-$C_{2-7}$-alkylester bilden auch Säureadditionssalze. Diese werden vorzugsweise mit solchen Säuren, welche therapeutisch verwendbare Säureadditionssalze ergeben, hergestellt. Säuren, die therapeutisch verwendbare Säureadditionssalze ergeben, sind z.B. anorganische Säuren, wie Halogenwasserstoffsäuren, z.B. Chlorwasserstoff- oder Bromwasserstoffsäure, oder Schwefel-, Phosphor-, Salpeter- oder Perchlorsäure; oder vorzugsweise organische Säuren, wie aliphatische oder aromatische Carbon- oder Sulfonsäuren, z.B. Ameisen-, Essig-, Propion-, Bernstein-, Glykol-, Milch-, Äpfel-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Äthansulfon-, Hydroxyäthansulfon-, Äthylensulfon-, Halogenbenzolsulfon-, Toluolsulfon-, Naphthalinsulfon-, Sulfanil- oder Cyclohexylsulfaminsäure; oder die Ascorbinsäure.

Die Verbindungen der Erfindung zeigen wertvolle pharmakologische Eigenschaften, in erster Linie hypotensive, anthihypertensive und bradykardische Wirkungen, welche sie unter anderem aufgrund ihrer Hemmwirkung auf das Enzym, welches das Angiotensin umwandelt, hervorrufen. Diese pharmakologischen Eigenschaften können durch in vivo oder in vitro Tierversuche, vorzugsweise an Säugetieren, wie Ratten, Katzen, Hunden oder ihren isolierten Organen, als Testobjekte nachgewiesen werden. Die Tiere können entweder normotensiv oder hypertensiv, z.B. genetisch hypertensive Ratten, oder renal hypertensive Ratten oder Hunde, und Hunde, denen Natrium entzogen worden ist, sein. Die genannten Verbindungen können ihnen enteral oder parenteral, vorzugsweise oral oder intravenös, z.B. durch Gelatine-Kapseln oder in Form von Stärke enthaltenden Suspensionen bzw. wässerigen Lösungen, verabreicht werden. Die verwendete Dosis kann in einem Bereich von 0,01 bis 50 mg/kg/Tag, vorzugsweise 0,1 bis 25 mg/kg/Tag, insbesondere 1 bis 10 mg/kg/Tag liegen.

Die in vivo blutdrucksenkende Wirkung entweder direkt mit einem Katheter, der z.B. in die femurale Arterie eines Hundes eingeführt ist, oder direkt durch Sphygmomanometrie am Rattenschwanz, und einem Übertragungsinstrument registriert. Der Blutdruck wird vor und nach der Verabreichung des Wirkstoffes in mmHg bestimmt. So sind z.B. die repräsentativen Verbindungen dieser Erfindung, welche in den Beispielen illustriert werden, in hypertensiven Ratten oder Hunden bereits bei einer Verabreichung in niedrigen oralen Dosen von 10 mg/kg/Tag oder darunter, sehr wirksam.

Die neuen Verbindungen zeigen auch eine Hemmwirkung auf das Ansprechen des Blutdrucks von normotensiven Ratten auf Angiotensin I. Das Enzym Renin verursacht unter normalen Verhältnissen eine spezifische Hydrolyse des zirkulierenden Renin-Substrat-Proteins. Diese Hydrolyse ergibt das Angiotensin I, welches durch die Einwirkung des genannten Enzyms, welches das Angiotensin umwandelt, in das stark gefässverengende Angiotensin II weiter hydrolysiert wird. Durch Hemmung des genannten Enzyms wird die Entstehung des Angiotensins II aus I verhindert. Diese Hemmung dämpft daher jedes Ansprechen des Blutdrucks nach einer Angiotensin-I-Attacke.

Der entsprechende in vivo Test wird mit männlichen, normotensiven Ratten, welche mit 100–120 mg/kg intraperitoneal verabreichtem Natriumsalz des Äthyl-(1-methylpropyl)-malonylthioharnstoffs narkotisiert sind, vorgenommen. Eine femurale Arterie und eine Wadenvene werden mit je einer Hohlnadel für die direkte Messung des Blutdrucks und für die intravenöse Verabreichung des Angiotensins I und der erfindungsgemässen Verbindungen, versehen. Nach der Stabilisierung des basalen Blutdrucks wird die Druckänderung nach drei, in 5minutigen Intervallen durchgeführten, Reizungen mit 0,33 µg/kg Angiotensin I i.v., bestimmt. Druckänderungen werden auch 5, 10, 15, 30 und 60 Minuten nach entweder intravenöser oder peroraler Verabreichung (mit Magensonde) der zu prüfenden Verbindungen bestimmt. Die letztgenannten Druckänderungen werden mit den Anfangswerten verglichen. Jede festgestellte Abnahme vom Ansprechen des Blutdrucks ist ein Hinweis auf die Hemmung des Enzyms, welches das Angiotensin umwandelt. Diese Abnahme kann bis zu 80%, nach Dosen von 10 mg/kg i.v. oder 50 mg/kg p.o., reichen und bis 60 Minuten aufrechterhalten werden.

Die in vitro Hemmung des Enzyms, welches das Angiotensin umwandelt, kann bei den Verbindungen dieser Erfindung analog zu Biochim. Biophys. Acta 293, 451 (1973) nachgewiesen werden. Gemäss dieser Methode werden die genannten Verbindungen in ungefähr 1 mMol Konzentrationen in Phosphatpuffer, welcher von aussen mit Eis gekühlt wird, aufgelöst. Zu diesen Lösungen gibt man zuerst verschiedene µl-Mengen von 1 mMolarem Histidyl-leucin in Phosphatpuffer und dann 100 µl von 5 mMolarem Hippuryl-histidyl-leucin in Phosphatpuffer und 50 µl des Angiotensin-umwandelnden Enzyms. Dieses Enzym wird aus Lungen von erwachsenen männlichen Kaninchen in Tris-Puffer, der Kalium- und Magnesiumchlorid und auch Rohrzucker enthält, frisch hergestellt. Die genannten Lösungen werden 30 Minuten bei 37° inkubiert und die weitere Reaktion wird durch Hinzufügen von 0,75 ml einer 0,6-normalen wässerigen Natriumhydroxidlösung gestoppt. Dann gibt man bei Zimmertemperatur 100 µl o-Phthalaldehyd und nach 10 Minuten 100 µl 6-normaler Chlorwasserstoffsäure dazu. Die Proben werden in einem Spektrophotometer bei 360 nm mit Wasser verglichen und ihre optische Dichte wird bestimmt. Diese Werte werden durch einen Konversionsfaktor einer Standard-Kurve angepasst, wobei man die während der genannten Inkubation von 30 Minuten gebildete Histidyl-leucin-Menge in Nanomolen erhält. Die Ergebnisse werden, um den $IC_{50}$-Wert zu bestimmen, gegenüber den Wirkstoff-Konzentrationen graphisch als Kurve dargestellt. Dieser Wert bedeutet diejenige Wirkstoff-Konzentration, welche die Hälfte der Aktivität einer Kontrollprobe, die keinen Wirkstoff enthält, ergibt. Die genannten repräsentativen Verbindungen der vorliegenden Erfindung sind auch in diesem in vitro Testsystem, bis zu dem $IC_{50}$-Wert von 39 nM und darunter, sehr wirksam.

Dementsprechend sind die Verbindungen der Erfindung wertvolle antihypertensive Mittel, insbesondere für die Herabsetzung von hohem Blutdruck (ohne Rücksicht auf die Ätiologie) und/oder Herzerkrankungen wie Herzinsuffizienz, und/oder anderen Zuständen, die mit Ödemen oder Ascites einhergehen, z.B. Leberzirrhose. Sie können auch als Zwischenprodukte zur Herstellung von anderen wertvollen Produkten, insbesondere von pharmakologisch wirksamen Präparaten eingesetzt werden.

Besonders bevorzugt sind Verbindungen der Formel I, worin R Wasserstoff, Methyl, Methoxy, Fluor, Chlor oder Trifluormethyl, vorzugsweise in 5-Stellung, bedeutet, jedes der Symbole m und p für 1 steht, q die Zahl 1 oder 2 bedeutet, und R′ für Wasserstoff oder Phenyl steht; ihre Mono- oder Bis-amide, die Mono- oder Bis-($C_{1-7}$-alkyl oder ω-amino-$C_{2-7}$alkyl)-ester, und Salze, insbesondere therapeutisch verwendbare Alkalimetall- oder Ammoniumsalze der genannten Säuren oder Säureadditionssalze der genannten Aminoalkylester.

Die Verbindungen der vorliegenden Erfindung werden nach an sich bekannten Methoden, vorzugsweise dadurch hergestellt, dass man

a) eine Verbindung der allgemeinen Formel II

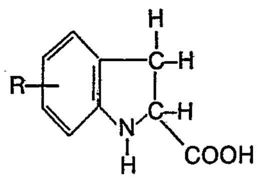

(II)

oder ihre genannten Säure- oder Amino-Derivate, mit einem reaktionsfähigen funktionellen Derivat einer Verbindung der allgemeinen Formel III

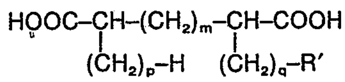

(III)

kondensiert, oder

b) eine Verbindung der allgemeinen Formel IV

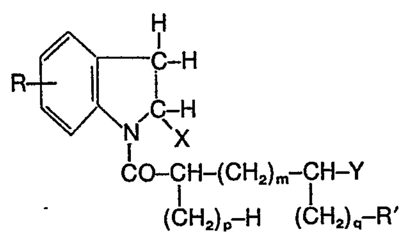

(IV)

worin mindestens eines der Symbole X und Y Cyan bedeutet, und das andere die genannte freie, amidierte oder veresterte Carboxygruppe ist, hydrolysiert oder alkoholysiert, oder

c) in einer Verbindung der allgemeinen Formel V

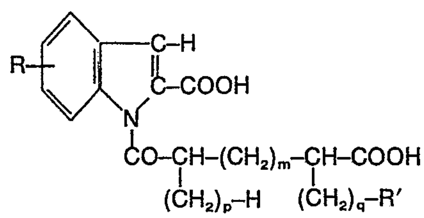

(V)

oder in ihren genannten Säure- oder Amino-Derivaten, den Indolteil zum Indolinteil hydriert, und, wenn erwünscht, eine erhaltene Verbindung in eine andere Verbindung der Erfindung umwandelt.

Reaktionsfähige funktionelle Derivate von Verbindungen der Formel III sind vorzugsweise Esterhalogenide, einfache oder gemischte Anhydride, z.B. die $C_{1-7}$-alkyl-halbester der genannten Säurechloride, das cyclische Anhydrid, oder gemischte Essigsäure- oder Cyanessigsäureanhydride. Die genannte Kondensation von Verbindungen II und III verläuft entweder spontan, oder in Gegenwart von Kondensationsmitteln, z.B. organischen oder anorganischen Basen, wie salzbildenden Aminen oder Metallcarbonaten, oder disubstituierten Carbodiimiden.

Die Hydrolyse der Nitrile IV zu den entsprechenden Säuren oder Amiden wird in an sich bekannter Weise, vorzugsweise mit anorganischen Säuren, z.B. Chlorwasserstoff- oder Schwefelsäure, durchgeführt. Die genannte Alkoholyse wird analog, in Gegenwart von den genannten Säuren und den entsprechenden unsubstituierten oder substituierten $C_{1-7}$-Alkanolen, vorgenommen.

Schliesslich wird die genannte Hydrierung von Indolen V zu den Indolinen I auch gemäss den konventionellen Hydrierungen von 1-Acylindolen, z.B. mit katalytisch aktiviertem oder nascierendem Wasserstoff, wie Wasserstoff in Gegenwart von Platin-, Palladium-, Rhodium- oder Nickel-Katalysatoren, oder mit elektrolytisch erzeugtem Wasserstoff, oder durch Einwirkung von Metallen auf Säuren oder Alkohole, durchgeführt. Auch Reduktionsmittel, z.B. einfache oder komplexe Leichtmetallhydride, wie Borane, oder vorzugsweise Alkalimetallborhydride oder -cyanborhydride, können verwendet werden. Bevorzugt ist die asymmetrische Hydrierung zu den Indolin-2S-carbonsäuren oder ihren genannten Derivaten. Man verwendet dabei chirale Katalysatoren, z.B. solche, die man aus einem Rhodiumsalz mit (R)-1,2-Bis-(diphenylphosphino)-propan oder (R)-1,2-Bis-(o-anisylphenylphosphino)-äthan und 1,5-Cyclooctadien herstellt.

Die erhaltenen Verbindungen der Erfindung können in an sich bekannter Weise in andere Verbindungen der Erfindung übergeführt werden. So können z.B. erhaltene Amide oder Ester weiter hydrolysiert oder alkoholysiert (umestert) werden. Dies kann gemäss dem Verfahren b) oder mit wässerigen Alkalien, z.B. mit Alkalimetall-carbonaten bzw. -hydroxiden, vorgenommen werden. Erhaltene freie Säuren können in an sich bekannter Weise, mit den genannten unsubstituierten oder substituierten $C_{1-7}$-Alkanolen oder Diazoalkanen verestert, oder in die genannten Metall-, Ammonium- oder in die Säureadditionssalze umgewandelt werden.

So kann z.B. eine erhaltene freie Säure oder Base in ein entsprechendes Metall-, Ammonium- bzw. Säureadditionssalz durch Umsetzung mit einer äquivalenten Menge einer entsprechenden Base, eines basischen Salzes, einer Säure oder eines Ionenaustauschers, umgewandelt werden. Die genannten Säuren werden dabei mit Alkalimetall- oder Ammoniumhydroxiden oder -carbonaten, und die genannten Aminoalkyl-ester mit den oben genannten anorganischen bzw. organischen Säuren behandelt. Ein erhaltenes Salz kann auch in die freie Verbindung, durch Behandlung mit stärkeren Säuren bzw. Basen übergeführt werden.

Infolge der engen Beziehung zwischen den neuen Verbindungen in freier Form und in Form ihrer Salze sind im vorausgegangenen und nachfolgend unter freien Verbindungen (oder Zwischenprodukten) und Salzen sinn- und zweckgemäss gegebenenfalls auch die entsprechenden Salze bzw. freien Verbindungen zu verstehen.

Die Ausgangsstoffe der Formeln II und III sind bekannt oder können, falls sie neu sind, nach an sich bekannten Methoden, z.B. analog wie in den Beispielen beschrieben, hergestellt werden.

Ausgangsstoffe der Formel IV können auch gemäss konventionellen Methoden, z.B. durch Kondensation von den Formeln II und/oder III entsprechenden Nitrilen gemäss dem oben beschriebenen Verfahren a) erhalten werden.

Ausgangsstoffe der Formel V können auch nach an sich bekannten Methoden, z.B. durch Einführung des entsprechenden Acylrestes in einen Indol-2-carbonsäure-$C_{1-7}$-alkylester erhalten werden.

Erhaltene geometrische oder optische Isomerengemische von obigen Verbindungen der Formeln I bis V können nach an sich bekannten Methoden, z.B. durch fraktionierte Destillation, Kristallisation und/oder Chromatographie, in die einzelnen Isomeren getrennt werden. Racemische Produkte können in die optischen Antipoden, z.B. durch Trennung ihrer diastereomeren Salze, wie gemäss J. Org. Chem. 43, 3803 (1978), z.B. durch fraktionierte Kristallisation von d- und l-(Tartraten, Mandelaten, Camphersulfonaten oder l-Naphtyl-1-äthylisocyanaten), oder von d- oder l-(α-Methylbenzylammonium, Cinchonidin, Cinchonin, Chinin, Chinidin, Ephedrin, Dehydroabietylamin, Brucin oder Strychnin)-salzen, gespalten werden. Der bevorzugte Ausgangsstoff der Formel II ist sein 2S-optisches Isomere (Epimere).

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, alkalischen oder sauren Kondensations- oder anderen oben genannten Mitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird.

Im Verfahren der vorliegenden Erfindung werden vorteilhafterweise solche Ausgangsstoffe verwendet, welche zu den im vorstehenden als besonders wertvoll beschriebenen Verbindungen, insbesondere solchen der Formel Ia, führen und die folgenden chiralen Isomeren sind:

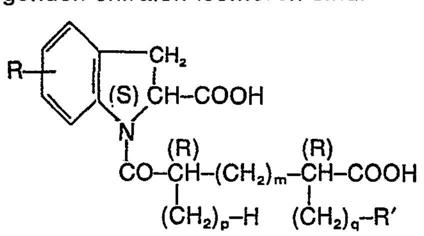

(Ia)

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannit, Sorbit, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, Enzyme der Bindemittel und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von 0,1 bis 75%, insbesondere von 1 bis 50% des Aktivstoffes. Einzeldosen für Säuger mit einem Gewicht von 50 bis 70 kg können zwischen 5 und 100 mg des aktiven Bestandteils enthalten.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 15 und 100 mm/Hg, durchgeführt.

Beispiel 1:

Eine Suspension von 5,0 g Indolin-2S-carbonsäureäthylesterhydrochlorid, 9,1 g pulverigem Kaliumcarbonat und 45 ml Methylenchlorid wird unter Rühren bei Zimmertemperatur mit 3,61 g Glutarsäuremethylester-chlorid versetzt. Das Gemisch wird über Nacht bei Zimmertemperatur gerührt, mit Eis gekühlt und mit 100 ml Wasser versetzt. Die organische Schicht wird abgetrennt, mit 1-normaler Chlorwasserstoffsäure und Wasser gewaschen, getrocknet und eingedampft. Man erhält den 1-(4-Carbomethoxybutanoyl)-indolin-2S-carbonsäure-äthylester, welcher bei 88–90° schmilzt.

Der Ausgangsstoff wird wie folgt hergestellt: Man löst 120 g 1-Acetylindolin-2-carbonsäure [Nippon Kagaku Zasshi 87, 760 (1966)] und 172 g l-Cinchonidin in 1200 g heissem Äthanol. Die Lösung wird über Nacht bei Zimmertemperatur und

dann 4 Tage bei 0° stehen gelassen. Das weisse kristalline Salz wird abfiltriert und weggeworfen. Das Filtrat wird eingedampft, mit 1000 ml Wasser versetzt und der pH-Wert der Lösung mit konzentrierter Chlorwasserstoffsäure auf 1 eingestellt. Nach 15 Minuten wird das Produkt durch Filtrieren abgetrennt, dreimal mit 250 ml 2-normaler Chlorwasserstoffsäure, zweimal mit 500 ml Wasser und zweimal mit 100 ml Äthanol gewaschen. Man erhält die 1-Acetylindolin-2S-carbonsäure, welche bei 214–215° schmilzt. $[\alpha]_D = -133{,}3°$ (c = 1,165 in Äthanol).

Eine Suspension von 37,5 g der letztgenannten Verbindung in 380 ml 2-normaler wässeriger Chlorwasserstoffsäure wird durch 5minütiges Durchperlen von Stickstoff von Sauerstoff befreit und dann 2 Stunden unter Rückfluss gekocht. Das Gemisch wird auf Zimmertemperatur gekühlt, durch Infusorienerde filtriert, das Filtrat eingedampft und der Rückstand aus Diäthyläther-Isopropanol umkristallisiert. Man erhält das Indolin-2S-carbonsäure-hydrochlorid, welches unter Zersetzung bei 133° schmilzt. $[\alpha]_D = -70{,}4°$ (c = 1 in Äthanol).

Eine Lösung von 34 g der letztgenannten Verbindung in 350 ml Äthanol wird ohne äussere Kühlung mit trockenem Chlorwasserstoff gesättigt. Das Gemisch wird zwei Stunden bei Zimmertemperatur gerührt und das Lösungsmittel bis zum Beginn der Kristallisation abgedampft. Das Konzentrat wird in 400 ml Diäthyläther gegossen, eine Stunde auf 0° gekühlt und filtriert. Man erhält das Indolin-2S-carbonsäure-äthylester-hydrochlorid, welches bei 179–181° schmilzt. $[\alpha]_D = -63°$ (c = 1,385 in Äthanol.

Beispiel 2:

Eine Suspension von 5,0 g 1-(4-Carbomethoxybutanoyl)-indolin-2S-carbonsäure-äthylester in 47 ml Methanol wird mit 47 ml 1-normaler wässeriger Natriumhydroxidlösung versetzt und das Gemisch 4 Stunden bei Zimmertemperatur gerührt. Das Reaktionsgemisch wird bei Zimmertemperatur und vermindertem Druck konzentriert und die wässerige Lösung unter Kühlen mit konzentrierter Chlorwasserstoffsäure angesäuert. Der erhaltene Niederschlag wird abgetrennt, mit Wasser gewaschen und getrocknet. Man erhält die 1-(4-Carboxybutanoyl)-indolin-2S-carbonsäure, welche bei 175–177° schmilzt. $[\alpha]_D = -97{,}8°$ (c = 1,0 in Äthanol).

Beispiel 3:

Eine Lösung von 11 g Indolin-2S-carbonsäure-hydrochlorid in 75 ml Pyridin wird mit 8,25 g 4-Carbomethoxy-2-methylbutanoyl-chlorid versetzt und das Gemisch über Nacht bei Zimmertemperatur gerührt. Das Pyridin wird bei Zimmertemperatur und vermindertem Druck abdestilliert, der Rückstand gekühlt, mit 3-normaler Chlorwasserstoffsäure angesäuert und mit Methylenchlorid extrahiert. Der Extrakt wird eingedampft, der Rückstand in 125 ml Diäthyläther gelöst und die Lösung mit 10 ml Dicyclohexylamin in 125 ml Hexan versetzt. Der Niederschlag wird abgetrennt, mit heissem Essigsäureäthylester gewaschen und über Nacht in Aceton suspendiert. Man erhält das Dicyclohexylammoniumsalz der 1-(4-Carbomethoxy-2-methylbutanoyl)-indolin-2S-carbonsäure, welches bei 203–205° schmilzt; die entsprechende freie 2S-Säure schmilzt bei 97–99°.

Der erste Ausgangsstoff ist im Beispiel 1 als Zwischenprodukt beschrieben. Der zweite kann wie folgt hergestellt werden:

Eine Lösung von 6,1 g 4-Carbomethoxy-2-methylbutansäure (US-A-4 052 511) in 50 ml Methylenchlorid wird mit 9,64 g Oxalylchlorid versetzt. Das Gemisch wird zwei Stunden unter Rückfluss gekocht und eingedampft. Man erhält das 4-Carbomethoxy-2-methyl-butanoylchlorid, welches ohne weitere Reinigung verwendet wird.

Beispiel 4:

Eine Lösung von 2,03 g Indolin-2S-carbonsäure-äthylester und 2,5 g 2-(2-Phenyläthyl)-glutarsäureanhydrid in 75 ml Toluol wird über Nacht in einer Stickstoffatmosphäre bei 70° erhitzt. Das Reaktionsgemisch wird eingedampft, der Rückstand in Diäthyläther gelöst, die Lösung mit 1-normaler Chlorwasserstoffsäure gewaschen und mit gesättigter wässeriger Natriumhydrogencarbonatlösung extrahiert. Der Extrakt wird gekühlt, mit Chlorwasserstoffsäure angesäuert und mit Methylenchlorid wieder extrahiert. Der organische Extrakt wird eingedampft, der Rückstand in Diäthyläther gelöst und die Lösung mit 1,2 ml Dicyclohexylamin in 25 ml Hexan versetzt. Der erhaltene Niederschlag wird abfiltriert und mit Hexan gewaschen. Man erhält das Dicyclohexylammoniumsalz des 1-[4-Carboxy-4-(2-phenyläthyl)-butanoyl]-indolin-2S-carbonsäure-äthylesters, welches bei 132–134° schmilzt. Diese Verbindung kann mit 1-normaler Chlorwasserstoffsäure in die freie Säure umgewandelt werden.

Der Ausgangsstoff kann wie folgt hergestellt werden:

Man lässt 29,8 g Indolin-2S-carbonsäure-äthylester-hydrochlorid sich zwischen 300 ml gesättigter wässeriger Natriumhydrogencarbonatlösung und 100 ml Methylenchlorid verteilen. Die wässerige Schicht wird zweimal mit weiteren 200 ml Methylenchlorid extrahiert. Die vereinigten organischen Schichten werden mit gesättigter wässeriger Natriumchloridlösung gewaschen und eingedampft. Man erhält den Indolin-2S-carbonsäure-äthylester als ein Öl, welches im IR-Spektrum die Hauptbande bei 1730 $cm^{-1}$ zeigt.

Eine Lösung von 12 g 2-(2-Phenyläthyl)-glutarsäure [J. Chem. Soc. 1950, 1683] in 75 ml Essigsäureanhydrid wird 4 Stunden unter Rückfluss gekocht und eingedampft. Der Rückstand wird aus Diäthyläther umkristallisiert. Man erhält das entsprechende Anhydrid, welches bei 78–80° schmilzt.

Beispiel 5:

Eine Lösung von 2,4 g 1-[4-Carboxy-4-(2-phenyläthyl)-butanoyl]-indolin-2S-carbonsäure-äthylester in 17,6 ml Methanol und 17,6 ml 1-normaler wässeriger Natriumhydroxidlösung wird 2,5 Stunden bei Zimmertemperatur gerührt. Das Reaktionsgemisch wird bei Zimmertemperatur unter vermindertem Druck konzentriert, die wässerige Lösung filtriert, mit Chlorwasserstoffsäure angesäuert und mit Methylenchlorid extrahiert. Der Extrakt wird eingedampft und der Rückstand aus Petroläther umkristallisiert. Man erhält die 1-[4-Carboxy-4-(2-phenyläthyl)-butanoyl]-indolin-2S-carbonsäure, welche bei 136–138° schmilzt.

Beispiel 6:

Gemäss den in den vorhergehenden Beispielen illustrierten Methoden werden auch die folgenden 1-(Carboxy-alkanoyl oder -aralkanoyl)-indolin-2S-carbonsäuren der Formel I, worin R = H, und auch ihre genannten Derivate hergestellt:

| No. | $-CH-(CH_2)_m-CH-$<br>$(CH_2)_p-H$ $(CH_2)_q-R'$ | $(CH_2)_p-H$ $(CH_2)_q-R'$<br>$-CH-(CH_2)_m-CH-COOH$<br>Derivat | Indolin-2-COOH Derivat | F. °C oder NMR |
|---|---|---|---|---|
| 1 | $-CH-CH_2-$<br>$CH_3$ | – | – | 122–124° 83–85° für Hemihydrat |
| 2 | $-(CH_2)_2-CH-$<br>$CH_3$ | – | Äthylester | 104–106° |
| 3 | $-(CH_2)_2-CH-$<br>$CH_3$ | – | – | 172–174° |
| 4 | $-CH-(CH_2)_2-$<br>$CH_3$ | Methylester | Äthylester | 4,25, 3,65 1,30 ppm |
| 5 | $-CH-(CH_2)_2-$<br>$CH_3$ | – | – | 72–74° |
| 6 | $-CH_2-CH-CH_2$<br>$CH_3$ | – | Äthylester | 111–113° |
| 7 | $-CH_2-CH-CH_2-$<br>$CH_3$ | – | – | 125–127° |
| 8 | $-CH-CH_2-CH-$<br>$CH_3$ $CH_3$<br>(erythro) | – | Äthylester-D | 132–134° |
| 9 | $-CH-CH_2-CH-$<br>$CH_3$ $CH_3$<br>(erythro) | – | – | 58–60° |
| 10 | $-CH-CH_2-CH-$<br>$CH_3$ $CH_3$<br>(threo) | – | – | 70–72° |
| 11 | $-CH-CH_2-CH-$<br>$CH_3$ $(CH_2)_2)-C_6H_5$<br>(erythro) | – | – | 1,17, 1,32 ppm |
| 12 | $-CH-CH_2-CH-$<br>$CH_3$ $(CH_2)_2)-C_6H_5$<br>(threo) | – | – | 1,15, 1,25 ppm |

D = Dicyclohexylammonium Salz

Die Ausgangstoffe für die genannten Verbindungen 1, 2, 4, 6, 11 und 12 sind das 3-Carbomethoxy-2-methylpropanoyl-chlorid, 2-Methyl-glutarsäure-anhydrid, 4-Carbomethoxy-2-methylbutanoylchlorid, 3-Methylglutarsäure-anhydrid und das erythro- bzw. threo-4-Carbomethoxy-4-(2-phenyläthyl)-2-methylbutanoyl-chlorid. Der Ausgangsstoff von Verbindungen 8 und 10 kann wie folgt hergestellt werden:

Eine Lösung von 6,0 g meso-2,4-Dimethylglutarsäure-anhydrid [J. Am. Chem. Soc. 77, 1862 (1955)] in 4 ml Methanol wird eine Stunde unter Rückfluss gekocht und eingedampft. Man erhält die erythro-4-Carbomethoxy-2,4-dimethylbutansäure. Sie wird mit 10,9 g Oxalylchlorid in 50 ml Methylenchlorid 2 Stunden unter Rückfluss gekocht und eingedampft. Man erhält das entsprechende Säurechlorid.

Das entsprechende threo-Isomere kann analog aus dem racemischen Anhydrid erhalten werden.

Beispiel 7:

Eine Lösung von 1 g 1-(4-Carbomethoxy-2-methylbutanoyl)-indolin-2S-carbonsäure (Beispiel 3) in 10 ml Methanol wird bei 0° mit Ammoniak gesättigt und in einer Druckflasche 4 Tage bei Zimmertemperatur gehalten. Es wird dann eingedampft, der Rückstand in Wasser aufgenommen, das Gemisch mit 2-normaler Chlorwasserstoffsäure bei 0° angesäuert. Die Kristallisation wird durch Hinzufügung von wenigen Tropfen Methylenchlorid ausgelöst. Das Gemisch wird filtriert und der Rückstand mit Diäthyläther trituiert. Man erhält die 1-(4-Carbamoyl-2-methylbutanoyl)-indolin-2S-carbonsäure, welche bei 192–194° schmilzt.

Beispiel 8:

a) Eine Lösung von 1,43 g Indolin-2S-carbonsäurehydrochlorid in 15 ml Pyridin wird bei 0° mit 1,35 g 4-Carboäthoxy-2R, 4R-dimethylbutanoylchlorid versetzt. Das Reaktionsgemisch wird bei Zimmertemperatur 3 Stunden gerührt und unter vermindertem Druck eingedampft. Der Rückstand wird mit 20 ml 3-normaler Chlorwasserstoffsäure behandelt und dreimal mit 10 ml Methylenchlorid extrahiert. Der Extrakt wird zur Trockene eingedampft. Die erhaltene 1-(4-Carboäthoxy-2R,4R-dimethylbutanoyl)-indolin-2S-carbonsäure wird in 75 ml Äther gelöst und mit 2,3 ml Dicyclohexylamin behandelt.

Man erhält das kristalline Dicyclohexylammoniumsalz. Dieses wird in einem Gemisch von 40 ml Essigsäureäthylester und 45 ml 5%iger wässeriger Kaliumbisulfatlösung 1 Stunde aufgeschlämmt. Die Essigsäureäthylester-Schicht wird abgetrennt, mit Wasser gewaschen, über Natriumsulfat getrocknet und zur Trockene eingedampft. Nach Umkristallisation aus Hexan erhält man die 1-(4-Carboäthoxy-2R,4R-dimethylbutanoyl)-indolin-2S-carbonsäure, welche bei 125–127° schmilzt $[\alpha]_D = -159°$ (c = 0,2 in Äthanol).

b) Verwendet man 4-Carboäthoxy-2R-methylbutanoyl-chlorid anstelle von 4-Carboäthoxy-2R,4R-dimethylbutanoyl-chlorid im obigen Verfahren, so wird die 1-(4-Carboäthoxy-2R-methylbutanoyl)-indolin-2S-carbonsäure erhalten. F. 133–135°. $[\alpha]_D = -120{,}5°$ (c = 0,2 in Äthanol).

c) Ähnlich wird auch die 1-(4-Carboäthoxy-2R-isopropylbutanoyl)-indolin-2S-carbonsäure hergestellt.

d) In analoger Weise werden auch die 1-(4-Carboäthoxy-2R,4R-dimethyl-butanoyl)-indolin-2S-carbonsäuren, in welchen die 5-Stellung des Indolinkernes durch Methoxy, Chlor oder Methyl substituiert ist, hergestellt.

Die Ausgangsstoffe werden wie folgt hergestellt:

Eine Lösung von 3,7 g 2R-Methylglutarsäure [J. Am. Chem. Soc. 77, 3383 (1955)] in 10 ml Acetylchlorid wird 2 Stunden bei 50° gerührt. Das Reaktionsgemisch wird zur Trockene eingedampft. Man erhält das 2R-Methylglutarsäure-anhydrid, welches bei 50–52° schmilzt. $[\alpha]_D = +43{,}8°$ (c = 1,0 in Chloroform). Das 2R,4R-Dimethylglutarsäureanhydrid, F. 43–45°, $[\alpha]_D = +56{,}5°$ (c = 1,0 in Chloroform) wird in analoger Weise ausgehend von 2R,4R-Dimethylglutarsäure (Arkiv Kemi Mineral. Geol. B 14, 1 (1940)], $[\alpha]_D$ $-35{,}5°$ (c = 2,0 in Äthanol) hergestellt.

In analoger Weise wird das 2R-Isopropylglutarsäure-anhydrid aus der entsprechenden 2R-Isopropylglutarsäure [Arkiv-Kemi Mineral. Geol. B 23, 1 (1946)] hergestellt.

Eine Lösung von 1,7 g 2R,4R-Dimethylglutarsäure-anhydrid in 40 ml absolutem Äthanol wird über Nacht unter Rückfluss gekocht und zur Trokkene eingedampft. Man erhält die 4-Carboäthoxy-2R,4R-dimethyl-butansäure als ein Öl $[\alpha]_D = -49{,}4°$ (c = 1,0 in Äthanol).

Eine Lösung von 2,9 g 2R-Methylglutarsäureanhydrid in 10 ml Äthanol wird 3 Stunden unter Rückfluss gekocht und zur Trockene eingedampft. Eine Lösung des erhaltenen Öls in 25 ml Äther wird mit 5,0 ml Dicyclohexylamin in 25 ml Hexan behandelt. Man erhält die 4-Carboäthoxy-2R-methylbutansäure als das Dicyclohexylammoniumsalz. F. 98–100°. Überführung in die freie Säure mit 1-normaler Chlorwasserstoffsäure und Extraktion mit Essigsäureäthylester ergibt die 4-Carboäthoxy-2R-methylbutansäure als ein Öl. $[\alpha]_D = -20{,}9°$ (c = 1,0 in Chloroform).

Eine Lösung von 1,27 g 4-Carboäthoxy-2R,4R-dimethylbutansäure in 15 ml Methylenchlorid wird mit 1,7 g Oxalylchlorid behandelt, 3 Stunden unter Rückfluss gekocht und zur Trockene eingedampft. Man erhält das 4-Carboäthoxy-2R,4R-dimethylbutanoyl-chlorid, welches NMR-Peaks bei 1,8, 2,5, 2,8 und 4,1 ppm zeigt. In analoger Weise werden das 4-Carboäthoxy-2R-methylbutanoyl-chlorid (NMR-Peaks bei 2,0, 2,4 und 4,3 ppm) und das 4-Carboäthoxy-2R-isopropyl-butanoyl-chlorid hergestellt.

Die 5-Methoxy-indolin-2-carbonsäure, 5-Chlorindolin-2-carbonsäure und die 5-Methyl-indolin-2-carbonsäure können gemäss der im Beispiel 1 beschriebenen Methode ausgehend von entsprechenden substituierten Indol-2-carbonsäuren hergestellt werden.

Beispiel 9:

a) Eine Lösung von 2,63 g Indolin-2S-carbonsäure-äthylester in 40 ml Methylenchlorid, welche 4,8 g wasserfreies Kaliumcarbonat enthält, wird mit 2,39 g 4-Carboäthoxy-2R,4R-dimethylbutanoyl-chlorid versetzt. Das Reaktionsgemisch wird über Nacht bei Zimmertemperatur gerührt und dann mit 20 ml Wasser extrahiert. Die organische Schicht wird mit 15 ml 1n-Chlorwasserstoffsäure und 15 ml Wasser gewaschen, über Natriumsulfat getrocknet und eingedampft. Man erhält den 1-(4-Carboäthoxy-2R,4R-dimethylbutanoyl)-indolin-2S-carbonsäure-äthylester als ein Öl. $[\alpha]_D$ = $-130,10°$ (c = 1,0 in Äthanol).

b) Verwendet man als Acylierungsmittel 4-Carboäthoxy-2R-methylbutanoyl-chlorid, so erhält man den 1-(4-Carboäthoxy-2R-methylbutanoyl)-indolin-2S-carbonsäure-äthylester als ein Öl, welches NMR-Peaks bei 1,2, 4,1 bis 4,3, 4,9, 6,7 und 7,2 ppm aufweist.

Beispiel 10:

Eine Lösung von 3,2 g Indolin-2S-carbonsäure-äthylester-hydrochlorid und 1,47 g Triäthylamin in 60 ml Methylenchlorid wird zuerst mit 2,34 g 4-Carboäthoxy-2R-methyl-butansäure und dann mit 2,97 g 1-(3-Dimethylaminopropyl)-3-äthylcarbodiimid-hydrochlorid versetzt. Das Reaktionsgemisch wird 3 Tage bei Zimmertemperatur gerührt und in Wasser gegossen. Die organische Schicht wird abgetrennt und nacheinander mit 30 ml 1-normaler Chlorwasserstoffsäure, 30 ml Wasser und 30 ml 10%iger wässeriger Natriumhydrogencarbonatlösung gewaschen. Die organische Schicht wird über Natriumsulfat getrocknet und zur Trockene eingedampft. Man erhält den 1-(4-Carboäthoxy-2R-methylbutanoyl)-indolin-2S-carbonsäure-äthylester, welche mit dem Produkt des Beispiels 9b) identisch ist.

Beispiel 11:

Eine Lösung von Indolin-2S-carbonsäure-äthylester und 0,30 g 2R,4R-Dimethylglutarsäureanhydrid in 10 ml Toluol wird 18 Stunden bei 70° gerührt. Das Reaktionsgemisch wird auf Zimmertemperatur gekühlt, zweimal mit 5 ml 1-normaler Chlorwasserstoffsäure gewaschen und zweimal mit 10 ml 5%iger wässeriger Natriumhydrogencarbonatlösung extrahiert. Die vereinigten Bicarbonatextrakte werden mit 4,0 ml 12-normaler Chlorwasserstoffsäure angesäuert und dreimal mit 10 ml Methylenchlorid extrahiert. Die vereinigten Methylenchloridextrakte werden über Natriumsulfat getrocknet und eingedampft. Man erhält den 1-(4-Carboxy-2R,4R-dimethyl-butanoyl)-indolin-2S-carbonsäure-äthylester als ein Öl. Es weist NMR-Peaks bei 1,0 bis 1,3, 4,15, 5,10, 7,2 und 8,4 ppm auf.

Beispiel 12:

a) Eine Lösung von 3,4 g 1-(4-Carboäthoxy-2R,4R-dimethylbutanoyl)-indolin-2S-carbonsäure-äthylester in 30 ml Äthanol wird mit 28,2 ml 1-normaler wässeriger Natriumhydroxidlösung versetzt. Das Reaktionsgemisch wird 4 Stunden bei Zimmertemperatur gerührt, eingedampft (um das Äthanol zu beseitigen) und mit 3,5 ml konzentrierter Chlorwasserstoffsäure angesäuert. Das Gemisch wird viermal mit 10 ml Methylenchlorid extrahiert. Die vereinigten Extrakte werden über Natriumsulfat getrocknet, zur Trockene eingedampft und aus Äther-Petroläther umkristallisiert. Man erhält die 1-(4-Carboxy-2R,4R-dimethylbutanoyl)-indolin-2S-carbonsäure, welche bei 132–134° schmilzt. $[\alpha]_D$ = $-144°$ (c = 1,0 in Äthanol).

Eine ähnliche Hydrolyse von 0,46 g 1-(4-Carboxy-2R,4R-dimethyl-butanoyl)-indolin-2S-carbonsäure-äthylester (Beispiel 10) und Umkristallisation des Produkts aus Wasser, ergibt das 1-(4-Carboxy-2R,4R-dimethylbutanoyl)-indolin-2S-carbonsäure-hydrat, welches bei 93–95° schmilzt. $[\alpha]_D$ = $-142,8°$ (c = 1,0 in Äthanol).

b) In analoger Weise ergibt die Hydrolyse von 1-(4-Carboäthoxy-2R-methylbutanoyl)-indolin-2S-carbonsäure-äthylester die 1-(4-Carboxy-2R-methylbutanoyl)-indolin-2S-carbonsäure. Nach Umkristallisation aus Äther schmilzt das Produkt bei 147–149°. $[\alpha]_D$ = $-125°$ (c = 0,2 in Äthanol).

In analoger Weise ergibt die Hydrolyse der 1-(4-Carboäthoxy-2R-methylbutanoyl)-indolin-2S-carbonsäure die entsprechende Di-säure, welche mit der oben isolierten 1-(4-Carboxy-2R-methylbutanoyl)-indolin-2S-carbonsäure identisch ist.

Beispiel 13:

Eine Lösung von 90 mg Indolin-2S-carbonsäure-hydrochlorid wird gemäss Beispiel 8 mit 92 mg 4-Carboäthoxy-2R-methyl-4R-phenyläthyl-butanoylchlorid in Pyridin behandelt. Man erhält die 1-(4-Carboäthoxy-2R-methyl-4R-phenyläthyl-butanoyl)-indolin-2S-carbonsäure in Form des Dicyclohexylaminsalzes. F. 146–149°. Das Salz wird in die freie Säure (z.B. gemäss der im Beispiel 8 beschriebenen allgemeinen Methode) umgewandelt. Man erhält die 1-(4-Carboäthoxy-2R-methyl-4R-phenyläthyl-butanoyl)-indolin-2S-carbonsäure, welche bei 95–98° schmilzt. $[\alpha]_D$ = $-117,5°$ (c = 0,88 in Chloroform).

Der Ausgangsstoff wird teilweise gemäss dem in Tetrahedron Letters 1980, 4233-6 beschriebenen allgemeinen Verfahren, wie folgt hergestellt:

Eine Lösung von 2,0 g L-Prolinol (US-A-3 935 280) in 50 ml Methylenchlorid wird mit 25 ml 1-normaler wässeriger Natriumhydroxidlösung versetzt. Das Reaktionsgemisch wird auf 0° gekühlt, mit 4,0 g 4-Phenyl-buttersäure-chlorid versetzt, zuerst 4 Stunden bei 0° und dann 1 Stunde bei Zimmertemperatur stark gerührt. Das Gemisch wird mit gleichem Volumen Methylenchlorid verdünnt und die Schichten werden getrennt. Die organische Phase wird mit 30 ml Wasser gewaschen und über Natriumsulfat/Kaliumcarbonat getrocknet und das Lösungsmittel abgedampft. Man erhält das N-(4-Phenylbutanoyl)-L-prolinol, welches IR-Peaks bei 3280 und 1605 $cm^{-1}$ aufweist. $[\alpha]_D^{25}$ = $-40,3$ (c = 1,42 in Methanol). Eine Lösung von 5,3 g R-(−)-3-Benzyloxy-2-methylpropanol [Helv. Chim. Acta 60, 925 (1977)] in 200 ml trockenem Tetrahydrofuran wird mit 20,5 g N-Methyl-N,N'-dicyclohexylcarbodiimidiumjodid

[Angew. Chem. Int. Ed. 11, 229 (1972)] unter Stickstoff versetzt und das Reaktionsgemisch 14 Stunden bei Zimmertemperatur gerührt. Das Lösungsmittel wird abgedampft und der Rückstand mit 20 ml Äther und 5 ml Pentan versetzt. Das erhaltene gelbe feste Material wird gesammelt und die Mutterlaugen werden auf 200 g Silicagel mit Pentan chromatographiert. Man erhält das S-(+)-3-Benzyloxy-2-methyl-propyljodid, welches einen $R_f$-Wert von 0,60 (9 : 1 Pentan: Äther auf Silicagel) aufweist. $[\alpha]_D^{25} = +11,1°$ (c = 1,11 in Methanol). 1,75 g N-(4-Phenylbutanoyl)-L-prolinol werden in 2 ml trockenem Tetrahydrofuran gelöst und tropfenweise zu einer Lösung von Lithiumdiisopropylamid (15,6 mMol) in 50 ml Tetrahydrofuran, in einer Stickstoffatmosphäre bei 0°, gegeben. Nach 30 Minuten bei 0° wird das Gemisch mit 2,03 g S-(+)-3-Benzyloxy-2-methyl-propyljodid in 2 ml trockenem Tetrahydrofuran tropfenweise versetzt. Das Reaktionsgemisch wird 5 Stunden bei 0° und 15 Stunden bei −15° gerührt, und bei 0° mit überschüssiger wässeriger Ammoniumchloridlösung gelöscht. Das Reaktionsgemisch wird mit 30 ml Äther verdünnt und die Schichten werden getrennt. Die organische Phase wird mit 16 ml 1-normaler Chlorwasserstoffsäure, 15 ml gesättigter wässeriger Natriumchloridlösung und 15 ml gesättigter wässeriger Natriumhydrogencarbonatlösung gewaschen und über Natriumsulfat getrocknet. Nach Eindampfen des Lösungsmittels erhält man ein Öl, welches nach Filtrieren durch 60 g Silicagel mit Essigsäureäthylester, das N-(R,R-5-Benzyloxy-4-methyl-2-phenyläthyl-pentanoyl)-L-prolinol ergibt. $R_f = 0,51$ (Essigsäureäthylester/Silicagel).

Eine Lösung von 2,0 g N-(R,R-5-Benzyloxy-4-methyl-2-phenyläthyl-pentanoyl)-L-prolinol in 50 ml 1-normaler äthanolischer Chlorwasserstoffsäure wird in einer Stickstoffatmosphäre 15 Stunden unter Rückfluss gekocht. Das Lösungsmittel wird abgedampft und der Rückstand auf 60 g Silicagel mit Pentan-Äther (2:1) chromatographiert. Man erhält den R,R-5-Benzyloxy-4-methyl-2-phenyläthyl-pentansäure-äthylester. $R_f = 0,37$ (9:1, Pentan: Äther/$SiO_2$), $[\alpha]_D^{25} = +2,85°$ (c = 1,10 in Athanol).

Eine Lösung von 0,6 g R,R-5-Benzyloxy-4-methyl-2-phenyläthyl-pentansäure-äthylester in 50 ml wasserfreiem Äthanol wird bei Zimmertemperatur und 2,7 Atmosphären, 3 Stunden über 0,5 g 5%igem Palladium-auf-Kohle Katalysator hydriert. Der Katalysator wird dann abfiltriert und das Lösungsmittel abgedampft. Man erhält den R,R-5-Hydroxy-4-methyl-2-phenyläthyl-pentansäureäthylester. $R_f = 0,36$ (1:1 Gemisch von Pentan und Äther).

Man löst 0,35 g R,R-5-Hydroxy-4-methyl-2-phenyläthyl-pentansäure äthylester in 15 ml trokkenem Dimethylformamid bei Zimmertemperatur unter Stickstoff. Die Lösung wird mit 2,5 g Pyridinium-dichromat versetzt und das Gemisch 15 Stunden bei Zimmertemperatur gerührt. Es wird dann in 150 ml Wasser gegossen und die Lösung mit 4mal 40 ml Äther extrahiert. Die ätherischen Extrakte werden zuerst mit 30 ml Wasser und dann dreimal mit 20 ml einer 1:1-Lösung von Natriumhydrogencarbonat: Kaliumcarbonat (pH = 10,5) gewaschen. Die basische Waschlösung wird mit konzentrierter Schwefelsäure auf den pH-Wert 2 eingestellt, wobei man die Temperatur zwischen 5 und 10° hält und viermal mit je 20 ml Äther extrahiert. Die vereinigten ätherischen Extrakte werden mit 20 ml gesättigter wässeriger Natriumchloridlösung gewaschen, über Natriumsulfat/Magnesiumsulfat getrocknet und eingedampft. Man erhält die 4-Carboäthoxy-2R-methyl-4R-(phenyläthyl)-butansäure, $R_f = 0,50$ (99:1:100-Gemisch von Äther-Essigsäure-Hexan). $[\alpha]_D^{25} = -4,91°$ (c = 1,24 in Äthanol).

Die Behandlung der 4-Carboäthoxy-2R-methyl-4R-(phenyläthyl)-butansäure mit Oxalylchlorid in Methylenchlorid ergibt das 4-Carboäthoxy-2R-methyl-4R-phenyläthyl-butanoylchlorid, welches ohne weitere Reinigung verwendet wird.

Beispiel 14:

a) Gemäss dem im Beispiel 10 beschriebenen Verfahren setzt man 79 mg Indolin-2S-carbonsäure-äthylester-hydrochlorid mit 96,5 mg 4-Carboäthoxy-2R-methyl-4R-(phenyläthyl)-butansäure (Beispiel 13) in Gegenwart von 0,05 ml Triäthylamin und 66,5 mg 1-(3-Dimethylaminopropyl)-3-äthyl-carbodiimid-hydrochlorid um. Man erhält den 1-(4-Carboäthoxy-2R-methyl-4R-phenyläthylbutanoyl)-indolin-2S-carbonsäure-äthylester. $R_f = 0,6$ (9:1-Gemisch von Chloroform-Methanol/$SiO_2$).

b) Eine Lösung von 87 mg des obigen Diesters in 4 ml Methanol wird bei Zimmertemperatur mit 0,2 ml 2,2-normaler wässeriger Kaliumhydroxidlösung und 1,5 ml Wasser versetzt und das Reaktionsgemisch 2 Stunden bei Zimmertemperatur gerührt. Das Gemisch wird in üblicher Weise (s. Beispiel 12) aufgearbeitet. Man erhält die 1-(4-Carboäthoxy-2R-methyl-4R-phenyläthylbutanoyl)-indolin-2S-carbonsäure, welche als das Dicyclohexylaminsalz gereinigt wird. F. 145–148°. Das Produkt ist mit demjenigen des Beispiels 13 identisch.

c) Eine Lösung von 0,28 g 1-(4-Carboäthoxy-2R-methyl-4R-phenethylbutanoyl)-indolin-2S-carbonsäure in 3 ml Methanol wird mit 2 ml 1-normaler wässeriger Lithiumhydroxidlösung versetzt. Das Reaktionsgemisch wird bei 55° 6 Stunden gerührt und dann eingedampft. Der Rückstand wird in 30 ml Wasser aufgenommen und mit 15 ml Diäthyläther gewaschen. Die wässerige Phase wird auf den pH-Wert 2 mit 1-normaler wässeriger Chlorwasserstoffsäure eingestellt und dreimal mit je 25 ml Methylenchlorid extrahiert. Die vereinigten organischen Extrakte werden über Magnesiumsulfat getrocknet und eingedampft. Der Rückstand wird aus Pentan umkristallisiert. Man erhält die 1-(4-Carboxy-2R-methyl-4R-phenyläthyl-butanoyl)-indolin-2S-carbonsäure. F 137–139°. $[\alpha]_D = -62°$ (c = 0,25 in Chloroform).

Beispiel 15:

Herstellung von 10 000 Tabletten mit einem Gehalt von je 5 mg der aktiven Substanz:

Bestandteile:

| | |
|---|---|
| 1-[4-Carboxy-4-(2-phenyläthyl)-butanoyl]-indolin-2S-carbonsäure | 50 g |
| Milchzucker | 1157 g |
| Maisstärke | 75 g |
| Polyäthylenglykol 6000 | 75 g |
| Talkpulver | 5 g |
| Magnesiumstearat | 18 g |
| Gereinigtes Wasser | q.s. |

Verfahren: Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten mit 6,4 mm Durchmesser, welche eine Bruchrille aufweisen, gepresst.

Beispiel 16:

Herstellung von 10 000 Kapseln mit einem Gehalt von je 10 mg der aktiven Substanz:

Bestandteile

| | |
|---|---|
| 1-(4-Carboäthoxy-2R,4R-dimethylbutanoyl)-indolin-2S-carbonsäure | 100 g |
| Milchzucker | 1800 g |
| Talkpulver | 100 g |

Verfahren: Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 3 werden mit je 200 mg des Gemisches mit einer Füllmaschine gefüllt.

In analoger Weise werden Tabletten oder Kapseln von anderen erfindungsgemässen Verbindungen, z.B. solchen, die in den weiteren Beispielen hier illustriert sind, hergestellt.

Beispiel 17:

Eine Lösung von 0,78 g Indolin-2S-carbonsäure-äthylester-hydrochlorid, 0,90 g 4R-Carboäthoxy-6-phenyl-hexansäure und 0,48 ml Triäthylamin in 40 ml Methylenchlorid wird mit 0,72 g 1-(3-Dimethylaminopropyl)-3-äthylcarbodiimid-hydrochlorid versetzt. Das Reaktionsgemisch wird 4 Tage bei Zimmertemperatur gerührt und dann mit 300 ml Diäthyläther versetzt. Das Gemisch wird mit 25 ml Wasser, 25 ml 2-normaler wässeriger Chlorwasserstoffsäure und 25 ml gesättigter wässeriger Natriumhydrogencarbonatlösung gewaschen. Die organische Schicht wird über Magnesiumsulfat getrocknet, und eingedampft. Man erhält den 1-(4-Carboäthoxy-4R-phenyläthyl-butanoyl)-indolin-2S-carbonsäure-äthylester. $[\alpha]_D = -64{,}3°$ (c = 0,85 in Chloroform).

Der Ausgangsstoff für das obige Verfahren wird wie folgt hergestellt:

Man gibt 8,10 g N-(4-Phenylbutanoyl)-L-prolinol in 50 ml Tetrahydrofuran zu 71 mMol Lithiumdiisopropylamid in 270 ml Tetrahydrofuran bei −20°. Nach einer Stunde wird das Gemisch mit 3,1 ml Allylbromid versetzt. Man hält das Reaktionsgemisch 1 Stunde bei −20° und verdünnt es dann mit 250 ml Diäthyläther. Die Schichten werden getrennt und die organische Phase wird mit 200 ml 0,5-normaler wässeriger Chlorwasserstoffsäure und 200 ml gesättigter wässeriger Natriumchloridlösung gewaschen, über Natriumsulfat getrocknet und eingedampft. Man erhält das N-(2R-Allyl-4-phenyl-butanoyl)-L-prolinol. $[\alpha]_D = -23{,}5°$ (c = 1 in Methanol).

Eine Lösung von 7,65 g N-(2R-Allyl-4-phenylbutanoyl)-L-prolinol in 350 ml 10%iger äthanolischer Schwefelsäure wird 10 Stunden unter Rückfluss gekocht. Das Lösungsmittel wird abgedampft und der Rückstand mit 100 ml Wasser zusammen mit 200 ml Diäthyläther ausgeschüttelt. Die Schichten werden getrennt und die organische Phase wird mit 50 ml gesättigter wässeriger Natriumhydrogencarbonatlösung gewaschen, über Natriumsulfat getrocknet und eingedampft. Man erhält den 2R-Allyl-4-phenyl-buttersäure-äthylester. $R_f = 0{,}42$ (9:1 Hexan-Äther Gemisch, auf Silicagel).

Man gibt bei 0° zu 22,2 ml 1-molarem Boran in Tetrahydrofuran 3,1 g 2-Methyl-2-buten. Das Gemisch wird zwei Stunden bei 0° gehalten und dann mit 4,7 g 2R-Allyl-4-phenyl-buttersäure-äthylester versetzt. Nach zwei Stunden gibt man 7,3 ml 3-normale wässerige Natriumhydroxidlösung und 7,3 ml 30%iges wässeriges Wasserstoffperoxid dazu. Nach 30 Minuten wird das Reaktionsgemisch mit 75 ml Diäthyläther verdünnt.

Die Schichten werden getrennt und die wässerige Schicht wird zweimal mit je 25 ml Diäthyläther extrahiert. Die vereinigten Ätherextrakte werden mit 25 ml 1%iger wässeriger Natriumcarbonatlösung und mit 25 ml gesättigter wässeriger Natriumsulfitlösung gewaschen. Die organische Phase wird über Magnesiumsulfat getrocknet und eingedampft. Man erhält den 5-Hydroxy-2R-phenyläthyl-pentansäure-äthylester, der NMR-Peaks bei 7,3, 4,25, 3,61, 1,55–2,78 und 1,27 ppm zeigt.

Man gibt zu 4,97 g 5-Hydroxy-2R-phenyläthylpentansäure-äthylester in 100 ml Dimethylformamid 37,4 g Pyridinium-dichromat. Das Reaktionsgemisch wird über Nacht bei Zimmertemperatur gerührt und dann in 300 ml Eiswasser gegossen. Das Gemisch wird fünfmal mit je 100 ml Diäthyläther extrahiert. Die vereinigten organischen Extrakte werden mit 100 ml 5%iger wässeriger Natriumcarbonatlösung gewaschen. Die wässerige Phase wird mit konz. Schwefelsäure auf den pH-Wert 2 eingestellt und dreimal mit je 100 ml Diäthyläther extrahiert. Die vereinigten Ätherextrakte werden über Magnesiumsulfat getrocknet und eingedampft. Man erhält die 4R-Carboäthoxy-6-phenyl-hexansäure. $[\alpha]_D = +4{,}32°$ (c = 1 in Methanol).

Beispiel 18:

Ein Gemisch von 1,10 g 1-(4-Carboäthoxy-4R-phenyläthyl-butanoyl)-indolin-2S-carbonsäure-äthylester und 5 ml Äthanol wird mit 1,07 ml 2,21-normaler wässeriger Kaliumhydroxidlösung versetzt. Das Reaktionsgemisch wird 1 Stunde bei Zimmertemperatur gerührt und dann eingedampft. Der Rückstand wird in 20 ml gesättigter wässeriger Natriumhydrogencarbonatlösung aufgenommen und mit 20 ml Diäthyläther gewaschen. Die wässerige Schicht wird mit 2-normaler wässeriger Chlorwasserstoffsäure auf den pH-Wert 1 eingestellt und zweimal mit je 25 ml Methylenchlorid extrahiert. Die vereinigten Methylenchloridextrakte werden über Magnesiumsulfat getrocknet und eingedampft. Man erhält die 1-(4-Carboäthoxy-4R-phenyläthyl-butanoyl)-indolin-2S-carbonsäure, welche bei 100–102° schmilzt. $[\alpha]_D = -83{,}4°$ (c = 1,13 in Chloroform.

Beispiel 19:

Ein Gemisch von 0,67 g 1-(4-Carboäthoxy-4R-phenyläthyl-butanoyl)-indolin-2S-carbonsäure in 8 ml Methanol wird mit 4,9 ml 1-normaler wässeriger Lithiumhydroxidlösung versetzt. Das Reaktionsgemisch wird bei 55° 8 Stunden gerührt und dann eingedampft. Der Rückstand wird in 30 ml gesättigter wässeriger Natriumhydrogencarbonatlösung aufgenommen und mit 25 ml Diäthyläther gewaschen. Die wässerige Schicht wird mit 2-normaler wässeriger Chlorwasserstoffsäure angesäuert und zweimal mit je 25 ml Methylenchlorid extrahiert. Die vereinigten Methylenchloridextrakte werden über Magnesiumsulfat getrocknet und eingedampft. Der Rückstand wird aus Pentan umkristallisiert. Man erhält die 1-(4-Carboxy-4R-phenyläthyl-butanoyl)-indolin-2S-carbonsäure, welche bei 132–135° schmilzt. $[\alpha]_D = 79{,}1°$ (c = 0,81 in Chloroform).

Beispiel 20:

Eine Lösung von 5,0 g Indolin-2S-carbonsäure-äthylester-hydrochlorid und 2,2 g Triäthylamin in 40 ml Toluol wird mit 3,1 g (2R,4R)-Dimethylglutarsäure-anhydrid versetzt. Das Reaktionsgemisch wird bei 80° 4 Stunden gerührt, dann auf Zimmertemperatur gekühlt und mit 10 ml 1-normaler Chlorwasserstoffsäure und 10 ml Wasser gewaschen. Die organische Schicht wird über Natriumsulfat getrocknet und eingedampft. Der Rückstand wird aus Diäthyläther-Hexan-umkristallisiert. Man erhält den 1-(4-Carboxy-2R,4R-dimethyl-butanoyl)-indolin-2S-carbonsäure-äthylester, der bei 110–112° schmilzt. $[\alpha]_D = -156{,}5°$ (c = 0,2 in Äthanol).

Beispiel 21:

Eine Lösung von 6,5 g 1-(4-Carboäthoxy-2-isopropyl-butanoyl)-indolin-2S-carbonsäure-äthylester in 50 ml Methanol wird mit 52 ml 1-normaler wässeriger Natriumhydroxidlösung versetzt. Das Reaktionsgemisch wird 2 Stunden bei Zimmertemperatur gerührt und dann das Methanol abgedampft. Der wässerige Rückstand wird mit 5 ml konz. Chlorwasserstoffsäure angesäuert und dreimal mit je 25 ml Methylenchlorid extrahiert. Die vereinigten organischen Extrakte werden über Natriumsulfat getrocknet und eingedampft. Der Rückstand wird aus Diäthyläther kristallisiert. Man erhält die 1-(4-Carboxy-2-isopropyl-butanoyl)-indolin-2S-carbonsäure, welche bei 184–186° schmilzt. $[\alpha]_D = -81°$ (c = 0,2 in Äthanol).

Der Ausgangsstoff wird wie folgt hergestellt: Eine Lösung von 14,0 g 2-Isopropyl-glutarsäure in 80 ml Acetylchlorid wird bei 50° 2 Stunden gerührt und eingedampft. Man erhält das 2-Isopropylglutarsäureanhydrid, welches NMR-Peaks bei 0,98, 1,89, 2,43 und 2,85 ppm aufweist.

Eine Lösung von 4,0 g 2-Isopropyl-glutarsäureanhydrid in 20 ml Äthanol wird 3 Stunden unter Rückfluss gekocht und dann eingedampft. Man erhält die 4-Carboäthoxy-2-isopropyl-buttersäure, die NMR-Peaks bei 4,76, 2,31, 1,84, 1,21 und 0,95 ppm zeigt.

Eine Lösung von 5,62 g Indolin-2S-carbonsäure-äthylester-hydrochlorid, 5,0 g 4-Carboäthoxy-2-isopropyl-buttersäure und 2,5 g Triäthylamin in 80 ml Methylenchlorid wird mit 5,21 g 1-(3-Dimethylaminopropyl)-3-äthylcarbodiimid-hydrochlorid versetzt. Das Reaktionsgemisch wird über Nacht bei Zimmertemperatur gerührt. Dann wird es mit 40 ml Wasser, 30 ml 1-normaler Chlorwasserstoffsäure und 30 ml gesättigter wässeriger Natriumhydrogencarbonatlösung gewaschen, getrocknet und eingedampft. Man erhält den 1-(4-Carboäthoxy-2-isopropyl-butanoyl)-indolin-2S-carbonsäure-äthylester, welcher NMR-Peaks bei 7,16, 6,78, 5,11, 4,22, 3,31, 2,31, 1,98, 1,22 und 0,95 ppm aufweist.

Beispiel 22:

Eine Lösung von 4,0 g Indolin-2S-carbonsäure-hydrochlorid und 2,0 g Triäthylamin in 40 ml Toluol wird mit 2,84 g 3,3-Dimethyl-glutarsäureanhydrid versetzt. Das Reaktionsgemisch wird bei 80° 4 Stunden gerührt und dann eingedampft. Der Rückstand wird zwischen 25 ml 1-normaler Chlorwasserstoffsäure und 25 ml Methylenchlorid verteilt. Die organische Schicht wird abgetrennt, mit 20 ml Wasser gewaschen und zweimal mit je 20 ml gesättigter wässeriger Natriumhydrogencarbonatlösung extrahiert. Die vereinigten Bicarbonatextrakte werden mit 5 ml konz. Chlorwasserstoffsäure angesäuert und zweimal mit je 25 ml Methylenchlorid extrahiert. Die organische Schicht wird über Natriumsulfat getrocknet und eingedampft. Der Rückstand wird aus Äther-Hexan umkristallisiert. Man erhält die 1-(4-Carboxy-3,3-dimethyl-butanoyl)-indolin-2S-carbonsäure, welche bei 132–133° schmilzt. $[\alpha]_D = -151°$ (c = 0,2 in Äthanol).

Beispiel 23:

a) Eine Lösung von 1,0 g 5-Chlorindolin-2-carbonsäure-hydrochlorid und 0,43 g Triäthylamin in 15 ml Toluol wird mit 0,55 g 2R,4R-Dimethylglutarsäureanhydrid versetzt. Das Reaktionsgemisch wird 3,5 Stunden bei 80° gerührt und dann eingedampft. Der Rückstand wird zwischen 20 ml 1-normaler Chlorwasserstoffsäure und 20 ml Methylenchlorid verteilt. Die Methylenchloridschicht wird mit 10 ml Wasser gewaschen und dann zweimal mit je 10 ml gesättigter wässeriger Natriumhydrogencarbonatlösung extrahiert. Die vereinigten Bicarbonatextrakte werden mit 2,5 ml konz. Chlorwasserstoffsäure angesäuert und dreimal mit je 10 ml Methylenchlorid extrahiert. Die organischen Extrakte werden über Natriumsulfat getrocknet und eingedampft. Der Rückstand wird in 7 ml Diäthyläther und 0,4 g Dicyclohexylamin gelöst. Das Produkt wird abfiltriert und aus Essigsäureäthylester umkristallisiert. Man erhält das Bis-dicyclohexylammoniumsalz der 1-(4-Carboxy-2R,4R-dimethyl-butanoyl)-5-chlorindolin-2-carbonsäure, welches bei 185–187° schmilzt. $[\alpha]_D = -26,50°$ (c = 0,2 in Äthanol).

b) Ähnlich wird auch das Bis-dicyclohexylammoniumsalz der 1-(4-Carboxy-2R,4R-dimethylbutanoyl)-5-methylindolin-2-carbonsäure hergestellt. F. 198–200°. $[\alpha]_D = +0,9°$ (c = 0,2 in Äthanol).

c) Auch die 1-(4-Carboxy-2R,4R-dimethyl-butanoyl)-5-methoxyindolin-2S-carbonsäure, F. 150–152°, $[\alpha]_D = -130°$ (c = 0,2 in Äthanol) wird ähnlich hergestellt.

Der Ausgangsstoff wird wie folgt hergestellt: Ein Gemisch von 20 g 5-Methoxyindol-2-carbonsäure (J. Chem. Soc., 1970, 865) und 200 ml Essigsäureanhydrid wird 2 Stunden unter Rückfluss gekocht und dann auf Zimmertemperatur gekühlt. Das Gemisch wird filtriert, das Filtrat eingedampft und der Rückstand mit 300 ml Wasser gerührt. Es wird überschüssiges Natriumhydrogencarbonat hinzugefügt, das Gemisch 3 Stunden gerührt und dann mit 200 ml Diäthyläther gewaschen. Die wässerige Schicht wird mit konz. Chlorwasserstoffsäure auf den pH-Wert 1 eingestellt und filtriert. Man erhält die 1-Acetyl-5-methoxyindol-2-carbonsäure, welche bei 173–175° schmilzt.

Man löst 16,5 g der letztgenannten Verbindung in 250 ml Äthanol und hydriert sie bei einer Atmosphäre in Anwesenheit von 1 g Platinoxid. Nach 2 Stunden wird das Gemisch filtriert, das Filtrat auf 100 ml konzentriert und über Nacht bei 0° stehen gelassen. Man erhält als farblose Substanz die 1-Acetyl-5-methoxyindolin-2-carbonsäure, welche bei 164–167° schmilzt.

Man lässt 6,0 g der letztgenannten Verbindung in 60 ml 2-normaler wässeriger Chlorwasserstoffsäure 2 Stunden unter Rückfluss kochen und dampft das Gemisch ein. Der Rückstand wird in 50 ml Isopropanol gelöst und mit Diäthyläther bis zur Trübung versetzt. Nach der Kühlung bei 0° wird der Niederschlag abfiltriert. Man erhält das 5-Methoxyindolin-2-carbonsäure-hydrochlorid, welches unter Zersetzung bei 90–92° schmilzt.

Andere substituierte Indolin-2-carbonsäuren werden in analoger Weise hergestellt: Das 5-Chlorindolin-2-carbonsäure-hydrochlorid, F. 165–167° und das 5-Methylindolin-2-carbonsäure-hydrochlorid, F. 171–173°.

Beispiel 24:

Eine Lösung von 1,5 g 4-Cyanbuttersäure, 3,0 g Indolin-2S-carbonsäure-äthylester-hydrochlorid und 1,35 g Triäthylamin in 75 ml Methylenchlorid wird mit 3,8 g 1-(3-Dimethylaminopropyl)-3-äthylcarbodiimidhydrochlorid versetzt. Das Reaktionsgemisch wird bei Zimmertemperatur drei Tage gerührt und dann mit 50 ml Wasser, 50 ml 2-normaler Chlorwasserstoffsäure und 50 ml gesättigter wässeriger Natriumhydrogencarbonatlösung gewaschen. Die organische Schicht wird über Magnesiumsulfat getrocknet und eingedampft. Man erhält den 1-(4-Cyanbutanoyl)-indolin-2S-carbonsäure-äthylester, der im IR-Spektrum Hauptbanden bei 2240, 1730 und 1660 $cm^{-1}$ zeigt.

Ein Gemisch von 2,3 g 1-(4-Cyanbutanoyl)-indolin-2S-carbonsäure-äthylester, 30 ml Tetrahydrofuran und 10 ml Wasser wird mit 8,8 ml 1-normaler wässeriger Lithiumhydroxidlösung versetzt. Das Reaktionsgemisch wird über Nacht bei Zimmertemperatur gerührt, dann mit 50 ml Wasser verdünnt und mit 50 ml Diäthyläther gewaschen. Die wässerige Schicht wird mit 10 ml 2-normaler Chlorwasserstoffsäure angesäuert und zweimal mit je 50 ml Diäthyläther extrahiert. Die vereinigten Extrakte werden über Magnesiumsulfat getrocknet und eingedampft. Man erhält die 1-(4-Cyanbutanoyl)-indolin-2S-carbonsäure, welche bei 98–100° schmilzt. $[\alpha]_D = -112,6°$ (c = 0,9 in Äthanol).

Eine Lösung von 2,6 g 1-(4-Cyanbutanoyl)-indolin-2S-carbonsäure in 50 ml Äthanol und 50 ml Äther wird bei 0° mit trockenem Chlorwasserstoffgas gesättigt. Das Reaktionsgemisch wird dann eine Stunde bei Zimmertemperatur gerührt, auf 0° gekühlt und mit 50 ml Wasser versetzt. Nach 10 Minuten wird das Gemisch unter vermindertem Druck eingedampft, der Rückstand in 50 ml Wasser aufgenommen und zweimal mit je 50 ml Äther extrahiert. Die vereinigten Ätherextrakte werden über Magnesiumsulfat getrocknet und eingedampft. Der Rückstand wird aus Äther-Hexan umkristallisiert. Man erhält den 1-(4-Carboäthoxybutanoyl)-indolin-2S-carbonsäure-äthylester, welcher bei 72–73° schmilzt. $[\alpha]_D = -81,7°$ (c = 0,35 in Äthanol).

Kardiovaskulare Pharmakologie von Verbindungen der Erfindung

Die Prüfung der Wirkung von Verbindungen ist gemäss den Methoden für die Auswertung der Hemmung des Enzyms, welches das Angiotensin umwandelt [angiotensin converting enzyme = ACE] durchgeführt worden. In der biochemischen Beurteilung der in vitro ACE-Hemmung (ACE inhibition = ACEI) wird die Aktivität einer Verbindung im Kaninchen-Lungengewebe bestimmt.

In der in vivo Prüfungsmethode wird zuerst durch Verabreichung des Angiotensins I (AI) an das Versuchstier eine Blutdrucksteigerung hervorgerufen. Es wird dann die Hemmung der einzelnen Verbindungen auf diese Blutdrucksteigerung gemessen.

I. Biochemische Testmethode

Es wurde ein Kaninchen-Lungengewebe-Präparat [(Das and Saffer, J. Biol. Chem. 250: 6762 (1975)] für die Bestimmung von ACE nach der

Methode von Cheung und Cushman [Cheung and Cushman, Biochim. Biophys. Acta 293, 451 (1973)] verwendet. In dieser Methode wird die Menge des Histidyl-leucins, welche aus einem synthetischen Substrat innerhalb einer Inkubation von 30 Minuten bei 37° freigesetzt wird, spektrophotometrisch bestimmt. Die $IC_{50}$-Werte der ACE-Hemmung werden dann graphisch berechnet. Der $IC_{50}$-Wert ist die Konzentration (in Mol) der getesteten Verbindung, welche die in Abwesenheit der Testsubstanz entstehende Menge des Histidyl-leucins um 50% herabsetzt.

II. Methode der Hemmung der durch Angiotensin I (AI) hervorgerufenen Blutdrucksteigerung, durch intravenöse Verabreichung der Testverbindung (% AI-Hemmung)

In diesen Untersuchungen wird wie oben beschrieben, je ein Katheter in eine femurale Arterie und in eine Wadenvene der Ratte eingeführt.

Der arterielle Druck wurde von dem Arterienkatheter aus registriert, während man Angiotensin I (AI) und die einzelnen .Testverbindungen durch den Venenkatheter injiziert hatte. Die Hemmung der durch Angiotensin I verursachten Blutdrucksteigerung ist in der Tabelle als Durchschnittswert 30 Minuten nach der Verabreichung von ausgewählten Dosen der jeweiligen Verbindung wiedergegeben. Dabei werden solche Dosierungen ausgewählt, so dass u.a. der $ID_{50}$ (i.v.)-Wert ermittelt werden kann.

Ergebnisse:

| Verbindung des Beispiels | In vitro ACEI $IC_{50}$ (M) | Hemmung der durch Angiotensin I (AI) verursachten Blutdrucksteigerung i.v. Dosis (mg/kg) | % AI Hemmungen |
|---|---|---|---|
| 2 | $3\times10^{-7}$ | 10 | 77 |
| 6/5 | $6\times10^{-8}$ | 10 | 70 |
| 5 | $4\times10^{-8}$ | 10 | 71 |
| 12/a | $3\times10^{-8}$ | $ID_{50}$:0.23 mg/kg | |
| 8/a | $6\times10^{-5}$ | $ID_{50}$:0.29 mg/kg | |
| 12/b | $4\times10^{-8}$ | 1.0 | 95 |
| | | 0.1 | 37 |
| 8/b | $1\times10^{-5}$ | 0.1 | 32 |
| 6/1 | $3\times10^{-7}$ | | |
| 13 | $1\times10^{-5}$ | 0.1 | 80 |
| 14/c | $5\times10^{-9}$ | 0.1 | 61 |
| 21 | $5\times10^{-8}$ | 1.0 | 80 |
| 23/c | $1\times10^{-7}$ | | |
| 23/b | $5\times10^{-7}$ | | |
| 23/a | $4\times10^{-6}$ | | |

**Patentansprüche für die Vertragsstaaten BE, CH, DE, FR, IT, U, W, NL, SE**

1. Verbindungen der allgemeinen Formel I

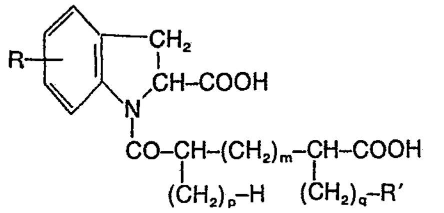

(I)

worin R Wasserstoff, $C_{1-4}$-Alkyl oder $C_{1-4}$-Alkoxy, Halogen oder Trifluormethyl bedeutet, m für die Zahl 0 oder 1 steht, jedes der Symbole p und q eine Zahl von 0 bis 2 bedeutet, und R′ für Wasserstoff oder Phenyl substituiert durch R steht; ihre Mono- oder Bisamide, die Mono- oder Bis-($C_{1-7}$-alkyl oder ω-amino-$C_{2-7}$-alkyl)-ester und ihre Salze.

2. Verbindungen der im Anspruch 1 gezeigten Formel I, in der Form von ihren 2S-Carboxy-indolin chiralen Epimeren.

3. 1-(4-Carboäthoxy-2R,4R-dimethylbutanoyl)-indolin-2S-carbonsäure und ihre Salze.

4. 1-(4-Carboxy-2R,4R-dimethylbutanoyl)-indolin-2S-carbonsäure und ihre Salze.

5. 1-(4-Carboäthoxy-2R-methyl-4R-phenyläthyl-butanoyl)-indolin-2S-carbonsäure und ihre Salze.

6. Pharmazeutische Präparate enthaltend Verbindungen der im Anspruch 1 gezeigten Formel I, ihre Mono oder Bis-amide, die Mono- oder Bis-($C_{1-7}$-Alkyl oder ω-amino-$C_{2-7}$alkyl)-ester und ihre therapeutisch verwendbaren Salze, zusammen mit einem pharmazeutischen Trägermaterial.

7. Die im Anspruch 1 genannten Verbindungen zur Anwendung in einem Verfahren zur therapeutischen Behandlung des menschlichen oder tierischen Körpers.

8. Die im Anspruch 1 genannten Verbindungen zur Verwendung als hypotensive, antihypertensive und bradykardische Mittel.

9. Verwendung der im Anspruch 1 genannten Verbindungen zur Herstellung von pharmazeutischen Präparaten.

10. Verfahren zur Herstellung von den im Anspruch 1 genannten Verbindungen der Formel I und ihren im Anspruch 1 genannten Derivaten, dadurch gekennzeichnet, dass man

a) eine Verbindung der allgemeinen Formel II

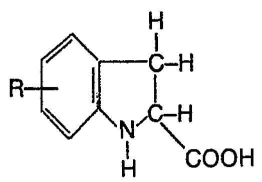

(II)

oder ihre genannten Säure- oder Amino-Derivate, mit einem reaktionsfähigen funktionellen Derivat einer Verbindung der allgemeinen Formel III

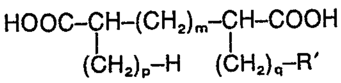

(III)

kondensiert, oder

b) eine Verbindung der allgemeinen Formel IV

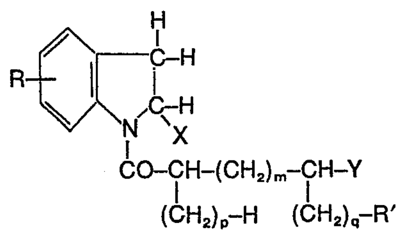

(IV)

worin mindestens eines der Symbole X und Y Cyan bedeutet, und das andere die genannte freie, amidierte oder veresterte Carboxygruppe ist, hydrolysiert oder alkoholysiert, oder

c) in einer Verbindung der allgemeinen Formel V

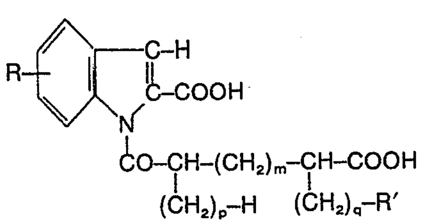

(V)

oder in ihren genannten Säure- oder Amino-Derivaten, den Indolteil zum Indolinteil hydriert, und, wenn erwünscht, eine erhaltene Verbindung in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomere oder Razemate auftrennt, und/oder, wenn erwünscht, erhaltene Razemate in die optischen Antipoden aufspaltet.

11. Die nach dem Verfahren des Anspruchs 10 erhältlichen Verbindungen.

12. Verbindungen, die nicht unter die allgemeine Formel I gemäss Anspruch 1 fallen, ausgewählt aus der Gruppe bestehend aus 1-(4Carboxy-3-methylbutanoyl)-indolin-2S-carbonsäureäthylester, 1-(4-Carboxy-3-methylbutanoyl)-indolin-2S-carbonsäure, 1-(4-Carboäthoxy-2R-isopropyl-butanoyl)-indolin-2S-carbonsäure, 1-(4-Carboxy-2-isopropyl-butanoyl)-indolin-2S-carbonsäure, und 1-(4-Carboxy-3,3-dimethyl-butanoyl)-indolin-2S-carbonsäure sowie deren Salze.

**Patentansprüche für den Vertragsstaat AT**

1. Verfahren zur Herstellung von 1-Carboxy-(alkanoyl oder aralkanoyl)-indolin-2-carbonsäuren der allgemeinen Formel I,

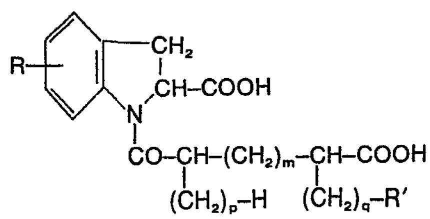

(I)

worin R Wasserstoff, $C_{1-4}$-Alkyl oder $C_{1-4}$Alkoxy, Halogen oder Trifluormethyl bedeutet, m für die Zahl 0 oder 1 steht, jedes der Symbole p und q eine Zahl von 0 bis 2 bedeutet, und R′ für Wasserstoff oder Phenyl substituiert durch R steht; ihren Mono- oder Bisamiden, Mono- oder Bis-($C_{1-7}$-alkyl oder ω-Amino-$C_{2-7}$-alkyl)-estern, und ihren Salzen, dadurch gekennzeichnet, dass man

a) eine Verbindung der allgemeinen Formel II

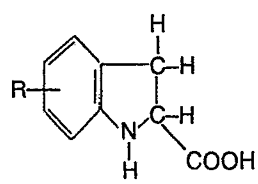

(II)

oder ihre genannten Säure- oder Amino-Derivate, mit einem reaktionsfähigen funktionellen Derivat einer Verbindung der allgemeinen Formel III

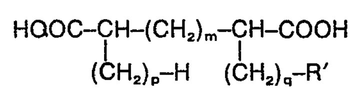

(III)

kondensiert, oder

b) eine Verbindung der allgemeinen Formel IV

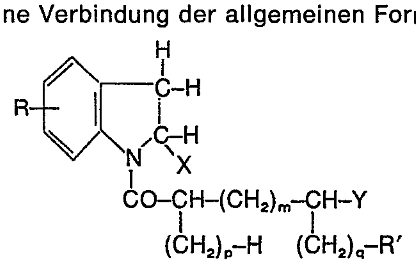

(IV)

worin mindestens eines der Symbole X und Y Cyan bedeutet, und das andere die genannte freie, amidierte oder veresterte Carboxygruppe ist, hydrolysiert oder alkoholysiert, oder

c) in einer Verbindung der allgemeinen Formel V

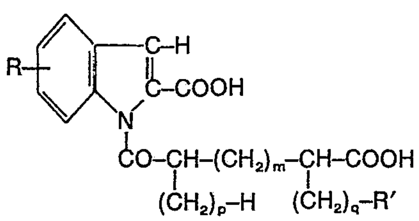

(V)

oder in ihren genannten Säure- oder Amino-Derivaten, den Indolteil zum Indolinteil hydriert, und, wenn erwünscht, eine erhaltene Verbindung in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

2. Verfahren nach Anspruch 1, dadurch gekennzeichnet, dass man Verbindungen der Formel I in der Form von ihren 2S-Carboxy-indolin chiralen Epimeren herstellt.

3. Verfahren nach Anspruch 1, dadurch gekennzeichnet, dass man 1-(4-Carboäthoxy-2R,4R-dimethylbutanoyl)-indolin-2S-carbonsäure und ihre Salze herstellt.

4. Verfahren nach Anspruch 1, dadurch gekennzeichnet, dass man 1-(4-Carboxy-2R,4R-dimethylbutanoyl)-indolin-2S-carbonsäure und ihre Salze herstellt.

5. Verfahren nach Anspruch 1, dadurch gekennzeichnet, dass man 1-(4-Carboäthoxy-2R-methyl-4R-phenyläthylbutanoyl)-indolin-2S-carbonsäure und ihre Salze herstellt.

6. Verfahren zur Herstellung eines pharmazeutischen Präparates, gekennzeichnet durch die Verarbeitung eines erfindungsgemässen Wirkstoffes nach einem der Ansprüche 1 oder 2 mit einem pharmazeutischen Trägermaterial.

7. Verfahren zur Herstellung eines pharmazeutischen Präparates, gekennzeichnet durch die Verarbeitung eines erfindungsgemässen Wirkstoffes nach einem der Ansprüche 3 bis 5 mit einem pharmazeutischen Trägermaterial.

8. Verfahren nach Anspruch 1, dadurch gekennzeichnet, dass man Verbindungen, die nicht unter die im Anspruch 1 angegebene allgemeine Formel I fallen, ausgewählt aus der Gruppe bestehend aus 1-(4-Carboxy-3-methyl-butanoyl)-indolin-2S-carbonsäureäthylester, 1-(4-Carboxy-3-methylbutanoyl)-indolin-2S-carbonsäure, 1-(4-Carboäthoxy-2R-isopropyl-butanoyl-indolin-2S-carbonsäure, 1-(4-Carboxy-2-isopropyl-butanoyl)-indolin-2S-carbonsäure, 1-(4-Carboxy-3,3-dimethyl-butanoyl)-indolin-2S-carbonsäure sowie deren Salze herstellt.

**Claims for the Contracting States: DE, FR, CH, LI, IT, NL, BE, SE, LU**

1. A compound of the general formula I

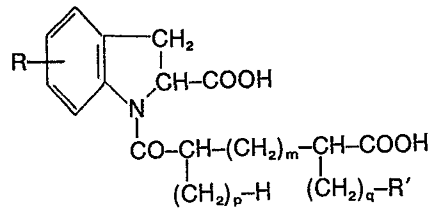

(I)

wherein R is hydrogen, $C_1$–$C_4$-alkyl or $C_1$–$C_4$-alkoxy, halogen or trifluoromethyl, m is 0 or 1, each of p and q is an integer from 0 to 2, and R′ is hydrogen or R-phenyl; or a mono- or bisamide, a mono- or bis($C_1$–$C_7$-alkyl or ω-amino-$C_2$–$C_7$-alkyl) ester or a salt thereof.

2. A compound of the formula I as indicated in claim 1, in the form of its 2S-carboxyindoline chiral epimer.

3. 1-(4-Carboethoxy-2R,4R-dimethylbutanoyl)-indoline-2S-carboxylic acid or a salt thereof.

4. 1-(4-Carboxy-2R,4R-dimethylbutanoyl)-indoline-2S-carboxylic acid or a salt thereof.

5. 1-(4-Carboethoxy-2R-methyl-4R-phenylethylbutanoyl)-indoline-2S-carboxylic acid or a salt thereof.

6. A pharmaceutical composition containing a compound of the formula I as indicated in claim 1, a mono- or bisamide thereof, a mono- or bis-($C_1$–$C_7$-alkyl or ω-amino-$C_2$–$C_7$-alkyl)ester thereof or a therapeutically acceptable salt thereof, together with a pharmaceutically acceptable carrier.

7. A compound as claimed in claim 1 for use in a therapeutic method of treating the human or animal body.

8. A compound as claimed in claim 1 for use as a hypotensive, antihypertensive or bradycardic agent.

9. Use of a compound as claimed in claim 1 for the preparation of pharmaceutical compositions.

10. A process for the preparation of a compound of the formula I as claimed in claim 1 or of a derivative thereof as claimed in claim 1, which process comprises

a) condensing a compound of the general formula II

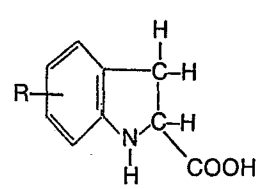

(II)

or an acid or amino derivative thereof, with a reactive functional derivative of a compound of the general formula III

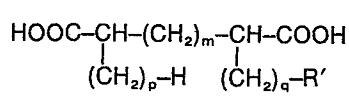

(III)

or

b) hydrolysing or alcoholysing a compound of the general formula IV

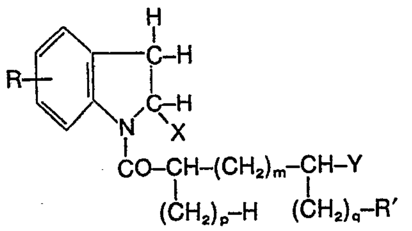

(IV)

wherein at least one of X and Y is cyano, and the other is a free, amidated or esterified carboxyl group, or

c) in a compound of the general formula V

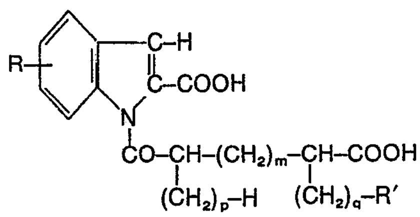

(V)

or in an acid or amino derivative thereof, hydrogenating the indole moiety to an indoline moiety, and, if desired, converting a resultant compound into another compound of the invention, and/or, if desired, converting a resultant free compound into a salt or a resultant salt into the free compound or into another salt, and/or, if desired, resolving a resultant mixture of isomere or racemates into the individual isomers or racemates, and/or, if desired, resolving a resultant racemate into the optical antipodes.

11. A compound obtainable by the process according to claim 10.

12. A compound not falling under the general formula I according to claim 1, selected from the group consisting of ethyl 1-(4-carboxy-3-methylbutanoyl)-indoline-2S-carboxylate, 1-(4-carboxy-3-methylbutanoyl)-indoline-2S-carboxylic acid, 1-(4-carboethoxy-2R-isopropylbutanoyl)-indoline-2S-carboxylic acid, 1-(4-carboxy-2-isopropylbutanoyl)-indoline-2S-carboxylic acid, 1-(4-carboxy-3,3-dimethylbutanoyl)-indoline-2S-carboxylic acid and salts thereof.

**Claims for the Contracting State: AT**

1. A process for the preparation of a 1-carboxy-(alkanoyl or aralkanoyl)indoline-2-carboxylic acid of the general formula I

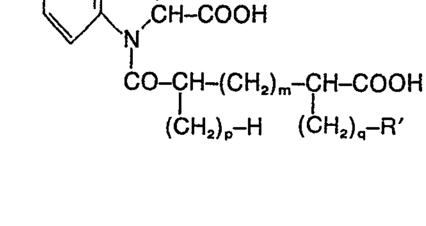

(I)

wherein R is hydrogen, $C_1$–$C_4$-alkyl or $C_1$–$C_4$-alkoxy, halogen or trifluoromethyl, m is 0 or 1, each of p and q is an integer from 0 to 2, and R′ is hydrogen or R-phenyl; or a mono- or bisamide, a mono- or bis-($C_1$–$C_7$-alkyl or ω-amino-$C_2$–$C_7$-alkyl) ester or a salt thereof, which process comprises

a) condensing a compound of the general formula II

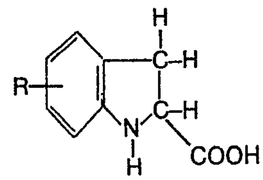

(II)

or an acid or amino derivative thereof, with a reactive functional derivative of a compound of the general formula III

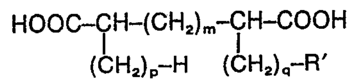

(III)

or

b) hydrolysing or alcoholysing a compound of the general formula IV

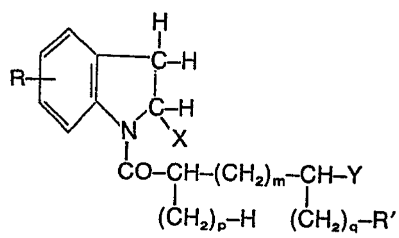

(IV)

wherein at least one of X and Y is cyano, and the other is a free, amidated or esterified carboxyl group, or

c) in a compound of the general formula V

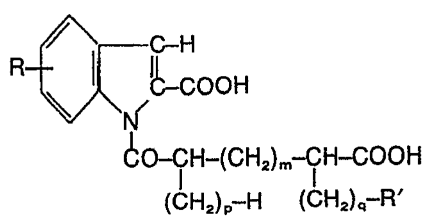

(V)

or in an acid or amino derivative thereof, hydrogenating the indole moiety to an indoline moiety, and, if desired, converting a resultant compound into another compound of the invention, and/or, if desired, converting a resultant free compound into a salt or a resultant salt into the free compound or into another salt, and/or, if desired, resolving a resultant mixture of isomere or racemates into the individual isomers or racemates, and/or, if desired, resolving a resultant racemate into the optical antipodes.

2. A process according to claim 1, which comprises preparing a compound of the formula I in the form of its 2S-carboxyindoline chiral epimer.

3. A process according to claim 1, which comprises preparing 1-(4-carboethoxy-2R,4R-dimethylbutanoyl)-indoline-2S-carboxylic acid or a salt thereof.

4. A process according to claim 1, which comprises preparing 1-(4-carboxy-2R,4R-dimethylbutanoyl)-indoline-2S-carboxylic acid or a salt thereof.

5. A process according to claim 1, which comprises preparing 1-(4-carboethoxy-2R-methyl-4R-phenylethylbutanoyl)-indoline-2S-carboxylic acid or a salt thereof.

6. A process for the preparation of a pharmaceutical composition, wherein a compound obtained according to either of claims 1 or 2 is combined with a pharmaceutically acceptable carrier.

7. A process for the preparation of a pharmaceutical composition, wherein a compound obtained according to any one of claims 3 to 5 is combined with a pharmaceutically acceptable carrier.

8. A process according to claim 1, which comprises preparing a compound not falling under the general formula I as indicated in claim 1, selected from the group consisting of ethyl
1-(4-carboxy-3-methylbutanoyl)-indoline-2S-carboxylate, 1-(4-carboxy-3-methylbutanoyl)-indoline-2S-carboxylic acid, 1-(4-carboethoxy-2R-isopropyl-butanoyl)-indoline-2S-carboxylic acid, 1-(4-carboxy-2-isopropyl-butanoyl)-indoline-2S-carboxylic acid,
1-(4-carboxy-3,3-dimethyl-butanoyl)-indoline-2S-carboxylic
acid and salts thereof.

**Revendications pour les Etats contractants: BE, CH, DE, FR, IT, LI, LU, NL, SE**

1. Composés de formule générale I:

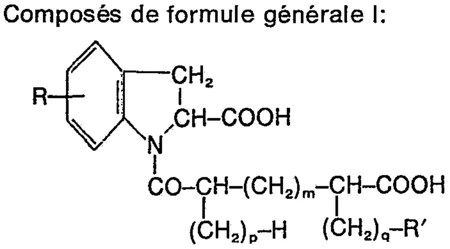

(I)

où R représente hydrogène, alcoyle en $C_1$ à $C_4$ ou alcoxy en $C_1$ à $C_4$, halogène ou trifluoro-méthyle, m représente le nombre 0 ou 1, chacun des symboles p et q représente un nombre allant de 0 à 2 et R′ représente un hydrogène ou un phényle substitué par R; leurs mono- ou bis-amides, les mono- ou bis-(alcoyle en $C_1$ à $C_7$ ou ω-amino-alcoyle en $C_2$ à $C_7$)-esters et leurs sels.

2. Composés de formule I présentés dans la revendication 1, sous la forme de leurs épimères chiraux de 2S-carboxy-indoline.

3. Acide 1-(4-carboéthoxy-2R,4R-diméthylbutanoyl)-indoline-2S-carboxylique et ses sels.

4. Acide 1-(4-carboxy-2R,4R-diméthylbutanoyl)-indoline-2S-carboxylique et ses sels.

5. Acide 1-(4-carboéthoxy-2R-méthyl-4R-phényléthyl-butanoyl)-indoline-2S-carboxylique et ses sels.

6. Préparations pharmaceutiques contenant des composés de formule I, présentés dans la revendication 1, leurs mono- ou bis-amides, les mono- ou bis-(alcoyle en $C_1$ à $C_7$ ou ω-amino-alcoyle en $C_2$ à $C_7$)-esters et leurs sels thérapeutiquement acceptables, avec un support pharmaceutiques.

7. Les composés mentionnés dans la revendication 1, aux fins d'application dans un procédé de traitement thérapeutique du corps humain ou animal.

8. Les composés mentionnés dans la revendication 1, aux fins d'application comme agents hypotenseurs, anti-hypertenseurs et bradycardiques.

9. Application des composés mentionnés dans la revendication 1, à la fabrication de préparations pharmaceutiques.

10. Procédé de préparation des composés de formule I mentionnés dans la revendication 1 et de leurs dérivés mentionnés dans la revendication 1, caractérisé en ce que:

a) on condense un composé de formule générale II

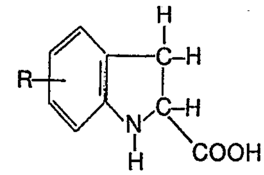

(II)

ou ses dérivés d'acide ou amino mentionnés, avec un dérivé fonctionnel réactif d'un composé de formule générale III

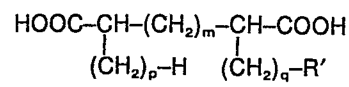

(III)

ou

b) on hydrolyse ou alcoolyse un composé de formule générale IV

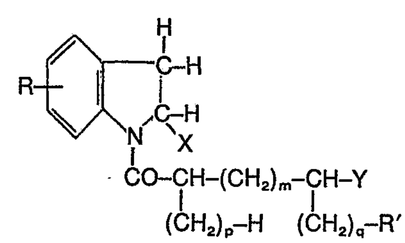

(IV)

où au moins un des symboles X et Y représente un cyano et l'autre est le groupe carboxy libre, amidé ou estérifié mentionné, ou

c) dans un composé de formule générale V

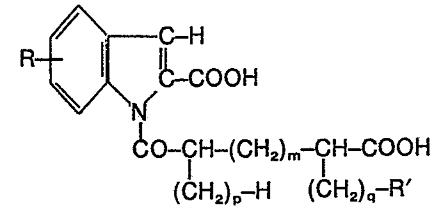

(V)

ou dans ses dérivés acide ou amino mentionnés, on nydrogène la fraction indole en fraction indoline et, si on le désire, on transforme le composé obtenu en un autre composé et/ou, si on le désire, on transforme un composé libre obtenu en un sel

ou un sel obtenu en composé libre ou en un autre sel et/ou, si on le désire, on dédouble un mélange d'isomères ou de racémates obtenu en isomères ou racémates isolés et/ou, si on le désire, on dédouble les racémates obtenus en antipodes optiques.

11. Les composés que l'on peut obtenir selon le procédé de la revendication 10.

12. Composés ne relevant pas de la formule générale I selon la revendication 1, choisis dans le groupe constitué par l'ester éthylique et l'acide 1-(4-carboxy-3-méthylbutanoyl)-indoline-2S-carboxylique, l'acide 1-(4-carboxy-3-méthylbutanoyl)-indoline-2S-carboxylique, l'acide 1-(4-carboéthoxy-2R-isopropyl-butanoyl)-indoline-2S-carboxylique, l'acide 1-(4-carboxy-2-isopropyl-butanoyl)-indoline-2S-carboxylique et l'acide 1-(4-carboxy-3,3-diméthyl-butanoyl)-indoline-2S-carboxylique et leurs sels.

**Revendications pour l'État contractant: AT**

1. Procédé de préparation d'acides 1-carboxy-(alcanoyl ou aralcanoyl)-indoline-2-carboxyliques de formule générale I

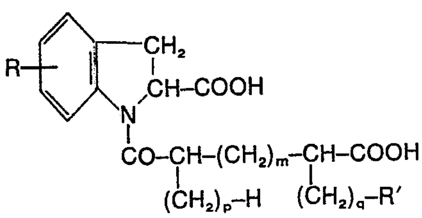

(I)

où R représente hydrogène, alcoyle en $C_1$ à $C_4$ ou alcoxy en $C_1$ à $C_4$, halogène ou trifluoro-méthyl, m représente le nombre 0 ou 1, chacun des symboles p et q représente un nombre allant de 0 à 2, et R' représente un hydrogène ou un phényle substitué par R; leurs mono- ou bis-amides, mono- ou bis-(alcoyle en $C_1$ à $C_7$ ou ω-amino-alcoyle en $C_2$ à $C_7$)-esters et leurs sels, caractérisé en ce que

a) on condense un composé de formule générale II

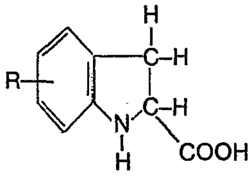

(II)

ou ses dérivés d'acide ou d'amino mentionnés, avec un dérivé fonctionnel réactif d'un composé de formule générale III

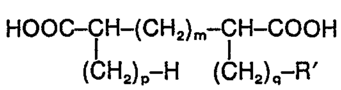

(III)

ou

b) on hydrolyse ou alcoolyse un composé de formule générale IV

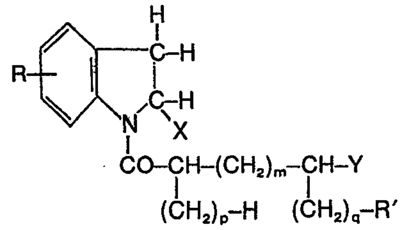

(IV)

où au moins un des symboles X et Y représente un cyano, et l'autre représente le groupe carboxy libre, amidé ou estérifié mentionné, ou

c) Dans un composé de formule générale V

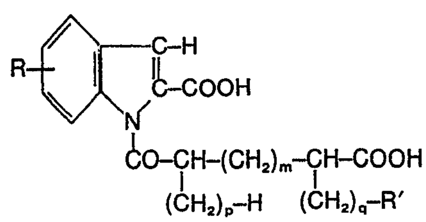

(V)

ou dans un de ses dérivés acide ou amino mentionnés, on hydrogène la fraction indole en fraction indoline et, si on le désire, on transforme un composé obtenu en un autre composé de l'invention et/ou, si on le désire, on transforme un composé libre obtenu en un sel ou un sel obtenu en composé libre ou en un autre sel et/ou, si on le désire, on dédouble un mélange d'isomères ou de racémates obtenu en isomères ou racémates isolés et/ou, si on le désire, on dédouble les racémates obtenus en antipodes optiques.

2. Procédé selon la revendication 1, caractérisé en ce qu'on prépare les composés de formule I sous la forme de leurs épimères chiraux de 2S-carboxy-indoline.

3. Procédé selon la revendication 1, caractérisé en ce qu'on prépare l'acide 1-(4-carboéthoxy-2R,4R-diméthylbutanoyl)-indoline-2S-carboxylique et ses sels.

4. Procédé selon la revendication 1, caractérisé en ce qu'on prépare l'acide 1-(4-carboxy-2R,4R-diméthyl-butanoyl)-indoline-2S-carboxylique et ses sels.

5. Procédé selon la revendication 1, caractérisé en ce qu'on prépare l'acide 1-(4-carboéthoxy-2R-méthyl-4R-phényléthylbutanoyl)-indoline-2S-carboxylique et ses sels.

6. Procédé de fabrication d'une préparation pharmaceutique, caractérisé par le traitement d'une substance active selon l'invention selon l'une des revendications 1 ou 2 avec un support pharmaceutique.

7. Procédé de fabrication d'une préparation pharmaceutique, caractérisé par le traitement d'une substance active selon l'invention d'une des revendications 3 à 5, avec un support pharmaceutique.

8. Procédé selon la revendication 1, caractérisé en ce qu'on prépare des composés qui ne relèvent

pas de la formule générale I donnés dans la revendication 1, choisis dans le groupe constitué par l'ester éthylique de l'acide 1-(4-carboxy-3-méthyl-butanoyl)-indoline-2S-carboxylique, l'acide 1-(4-carboxy-3-méthylbutanoyl)-indoline-2S-carboxylique, l'acide 1-(4-carboéthoxy-2R-isopropyl-butanoyl-indoline-2S-carboxylique, l'acide 1-(4-carboxy-2-isopropyl-butanoyl)-indoline-2S-carboxylique, l'acide 1-(4-carboxy-3,3-diméthyl-butanoyl)-indoline-2S-carboxylique ainsi que leurs sels.